(19) Europäisches Patentamt
European Patent Office
Office européen des brevets

(11) **EP 3 863 020 A1**

(12) **EUROPEAN PATENT APPLICATION**

(43) Date of publication:
**11.08.2021 Bulletin 2021/32**

(51) Int Cl.:
**G16C 20/80** (2019.01)  **G16C 60/00** (2019.01)

(21) Application number: **21152198.4**

(22) Date of filing: **18.01.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.02.2020   JP 2020018085**

(71) Applicant: **FUJITSU LIMITED**
**Kanagawa 211-8588 (JP)**

(72) Inventors:
• **KURITA, Tomochika**
**Kawasaki-shi, Kanagawa 211-8588 (JP)**
• **OHFUCHI, Mari**
**Kawasaki-shi, Kanagawa 211-8588 (JP)**

(74) Representative: **Haseltine Lake Kempner LLP**
**Cheapside House**
**138 Cheapside**
**London**
**EC2V 6BJ (GB)**

(54) **CRYSTALLINE MATERIAL ANALYSIS DEVICE, CRYSTALLINE MATERIAL ANALYSIS METHOD, AND CRYSTALLINE MATERIAL ANALYSIS PROGRAM**

(57)   A crystalline material analysis device includes: a graph creation unit that creates a loop graph that has intra-cell nodes that indicate atoms in one unit cell of a crystalline material, an intra-cell edge that indicates a chemical bond between atoms in the unit cell, and a loop edge that indicates the chemical bond, which is virtual, between atom X in the unit cell and atom Y in the unit cell corresponding to atom Y' in an adjacent unit cell that is adjacent to the unit cell, in which the atom Y' has the chemical bond with the atom X; and an analysis unit that analyzes the crystalline material by using the graph.

FIG. 10

EP 3 863 020 A1

**Description**

FIELD

The embodiments discussed herein are related to a crystalline material analysis device, a crystalline material analysis method, and a crystalline material analysis program useful for analysis of a crystalline material.

BACKGROUND

Computerized evaluation, prediction, inference, and the like are used to, for example, design a new chemical, predict characteristics of an existing chemical, and search for a new use for an existing chemical.

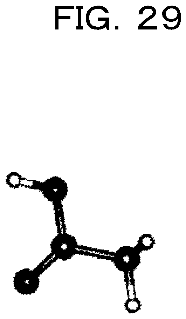

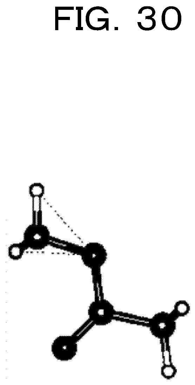

For example, in the field of organic chemistry, a structure of a molecule, for example, atoms and bonding relationships between the atoms are represented with a graph using nodes (spheres) and edges (rods) as illustrated in FIG. 29 (acetic acid) and FIG. 30 (methyl acetate). Then, a material search or design is performed by graph similarity evaluation using a graph, or by machine learning using a graph as input data.

However, in a case of a crystalline material, a unit cell is periodically repeated almost infinitely. It is therefore usually unable to determine a size of a graph for representing a crystalline structure. It is therefore not easy to represent a crystalline material with a graph and perform an analysis such as similarity evaluation.

Japanese Laid-open Patent Publication No. 2011-170444 is disclosed as related art.

It is desirable to provide a crystalline material analysis device, a crystalline material analysis method, and a crystalline material analysis program useful for analysis of a crystalline material.

SUMMARY

According to an aspect of the embodiments, a crystalline material analysis device includes: a graph creation unit that creates a loop graph that has intra-cell nodes that indicate atoms in one unit cell of a crystalline material, an intra-cell edge that indicates a chemical bond between atoms in the unit cell, and a loop edge that indicates the chemical bond, which is virtual, between atom X in the unit cell and atom Y in the unit cell corresponding to atom Y' in an adjacent unit cell that is adjacent to the unit cell, in which the atom Y' has the chemical bond with the atom X; and an analysis unit that analyzes the crystalline material by using the graph.

According to an aspect of the embodiments, it is possible to provide a crystalline material analysis device useful for analysis of a crystalline material.

Furthermore, according to an aspect of the embodiments, it is possible to provide a crystalline material analysis method useful for analysis of a crystalline material.

Furthermore, according to an aspect of the embodiments, it is possible to provide a crystalline material analysis program useful for analysis of a crystalline material.

BRIEF DESCRIPTION OF DRAWINGS

Embodiments are set out, by way of example only, with reference to the following drawings, in which:

FIG. 1 is a diagram illustrating a configuration example of a crystalline material analysis device of the present application;
FIG. 2 is a diagram illustrating another configuration example of the crystalline material analysis device of the present application;
FIG. 3 is a diagram illustrating another configuration example of the crystalline material analysis device of the present application;
FIG. 4 is a diagram illustrating an exemplary functional configuration as an embodiment of the crystalline material analysis device of the present application;
FIG. 5 is a diagram illustrating an exemplary functional configuration as another embodiment of the crystalline material analysis device of the present application;
FIG. 6 is a diagram illustrating an exemplary functional configuration as another embodiment of the crystalline material analysis device of the present application;
FIG. 7 is a schematic diagram illustrating an example of a crystalline structure of a crystalline material;
FIG. 8 illustrates an example of a graph of a unit cell;
FIG. 9 is a schematic diagram illustrating another example of a crystalline structure of a crystalline material;
FIG. 10 is a graph (loop graph) of a unit cell of the crystalline structure in FIG. 7, the graph being created by graphing according to the technique of the present application;

FIG. 11 is a graph (loop graph) of a unit cell of the crystalline structure in FIG. 9, the graph being created by graphing according to the technique of the present application;

FIG. 12 is a flowchart for analyzing a crystalline structure using an example of a first embodiment of the technique disclosed in the present application;

FIG. 13A is a flowchart of an example of a loop graph creation method (No. 1) ;

FIG. 13B is a flowchart of the example of the loop graph creation method (No. 2) ;

FIG. 13C is a flowchart of the example of the loop graph creation method (No. 3) ;

FIG. 14A is a diagram for illustrating an example of a comparison between a degree of similarity in a case of a conventional graph and a degree of similarity in a case of a loop graph in the first embodiment (No. 1);

FIG. 14B is a diagram for illustrating the example of the comparison between the degree of similarity in the case of the conventional graph and the degree of similarity in the case of the loop graph in the first embodiment (No. 2) ;

FIG. 14C is a diagram for illustrating the example of the comparison between the degree of similarity in the case of the conventional graph and the degree of similarity in the case of the loop graph in the first embodiment (No. 3) ;

FIG. 15A is a diagram for briefly illustrating an example of a second embodiment (No. 1);

FIG. 15B is a diagram for briefly illustrating an example of the second embodiment (No. 2);

FIG. 15C is a diagram for briefly illustrating an example of the second embodiment (No. 3);

FIG. 16A is a diagram for illustrating benefits of the second embodiment (No. 1) ;

FIG. 16B is a diagram for illustrating the benefits of the second embodiment (No. 2);

FIG. 17 is a flowchart for analyzing a crystalline structure using an example of the second embodiment of the technique disclosed in the present application;

FIG. 18A is a diagram for illustrating a working example of the second embodiment (No. 1);

FIG. 18B is a diagram for illustrating the working example of the second embodiment (No. 2);

FIG. 18C is a diagram for illustrating the working example of the second embodiment (No. 3);

FIG. 19A is a diagram for illustrating the working example of the second embodiment (No. 4);

FIG. 19B is a diagram for illustrating the working example of the second embodiment (No. 5);

FIG. 19C is a diagram for illustrating the working example of the second embodiment (No. 6);

FIG. 20A is a diagram for illustrating the working example of the second embodiment (No. 7);

FIG. 20B is a diagram for illustrating the working example of the second embodiment (No. 8);

FIG. 20C is a diagram for illustrating the working example of the second embodiment (No. 9);

FIG. 21A is a diagram for illustrating the working example of the second embodiment (No. 10);

FIG. 21B is a diagram for illustrating the working example of the second embodiment (No. 11);

FIG. 21C is a diagram for illustrating the working example of the second embodiment (No. 12);

FIG. 22A is a schematic diagram for illustrating replication of a graph in a third embodiment (No. 1);

FIG. 22B is a schematic diagram for illustrating replication of a graph in the third embodiment (No. 2);

FIG. 23A is a diagram for illustrating an example of how to determine the number of times a unit cell is to be replicated to create a host lattice (No. 1) ;

FIG. 23B is a diagram for illustrating an example of how to determine the number of times a unit cell is to be replicated to create a host lattice (No. 2) ;

FIG. 24 is a flowchart for analyzing a crystalline structure using an example of the third embodiment of the technique disclosed in the present application;

FIG. 25A is a diagram for illustrating a working example of the third embodiment (No. 1);

FIG. 25B is a diagram for illustrating the working example of the third embodiment (No. 2);

FIG. 25C is a diagram for illustrating the working example of the third embodiment (No. 3);

FIG. 26 is a conceptual diagram of graphs (simple graph, extension graph, and loop graph) formed from unit cell A;

FIG. 27 is a conceptual diagram of graphs (simple graph, extension graph, and loop graph) formed from unit cell B;

FIG. 28 is a conceptual diagram of an extension graph formed from unit cell A having edges between extension nodes;

FIG. 29 is a graph of acetic acid; and

FIG. 30 is a graph of methyl acetate.

## DESCRIPTION OF EMBODIMENTS

(Crystalline material analysis device, crystalline material analysis method, and crystalline material analysis program)

A crystalline material analysis device of the present application includes a graph creation unit and an analysis unit, and further includes a graph replication unit, a graph database, and the like depending on the situation.

A crystalline material analysis method of the present application includes performing, by a computer, a graph creation process and an analysis process, and further includes performing a graph replication process and the like depending on the situation.

A crystalline material analysis program of the present application causes a computer to perform graph creation and analysis, and to further perform graph replication and the like depending on the situation.

The crystalline material analysis device of the present application performs, for example, the crystalline material analysis method of the present application.

The crystalline material analysis method of the present application is performed by, for example, the crystalline material analysis program of the present application.

The crystalline material analysis device includes, for example, a memory and a processor, and further includes other units depending on the situation.

The processor is coupled to the memory.

The processor is configured to execute, for example, the graph creation process.

The processor is configured to execute, for example, the graph replication process.

The processor is configured to execute, for example, the analysis process.

The processor is, for example, a central processing unit (CPU), a graphics processing unit (GPU), or a combination thereof.

In the present application, a graph has a node, which is data on an atom, and an edge, which is data on a chemical bond between two atoms.

The graph has nodes (hereinafter sometimes referred to as "intra-cell nodes") indicating atoms in one unit cell of a crystalline material.

The graph has edges (hereinafter sometimes referred to as "intra-cell edges") indicating chemical bonds between atoms in the unit cell.

The graph may have an edge (hereinafter sometimes referred to as a "loop edge") indicating a virtual chemical bond between atom X in a unit cell and atom Y in the unit cell corresponding to atom Y' in an adjacent unit cell (hereinafter sometimes referred to simply as "adjacent unit cell") that is adjacent to the unit cell, in which the atom Y' has the chemical bond with the atom X in the unit cell. Here, the atom Y in the unit cell corresponding to the atom Y' in the adjacent unit cell means an atom in the unit cell that overlaps with the atom Y' in the adjacent unit cell when the adjacent unit cell and the unit cell are overlapped so that constituent atoms overlap.

The graph may have a node (hereinafter sometimes referred to as an "extension node") indicating an atom in the adjacent unit cell that is adjacent to the unit cell, in which the atom has a chemical bond with an atom in the unit cell.

The graph may have an edge (hereinafter sometimes referred to as an "extension edge") indicating a chemical bond between an atom corresponding to an intra-cell node and an atom corresponding to an extension node.

The graph may have an edge (hereinafter sometimes referred to as an "edge between extension nodes") indicating a chemical bond between two atoms corresponding to extension nodes.

The graph is stored in, for example, the graph database.

The graph creation unit creates a graph.

In the graph creation process, a graph is created.

The graph has a node, which is data on an atom, and an edge, which is data on a chemical bond between two atoms. The graph is an abstract data type constituted by a group of nodes (vertices) and a group of edges (lines) representing link relationships between the nodes. The graph is represented by G = (V, E), where V is a set of nodes and E is a set of edges. V is a finite set, and E is a set of sets constituted by two elements selected from V.

Intra-cell nodes and extension nodes have data such as an atom type, an atom valence, and an atom charge, for example.

Intra-cell edges, loop edges, and extension edges have data such as a bond type, a bond angle, a bond distance, and a bond order, for example. The bond angle and the bond distance are provided as coordinate data on, for example, an atom or a chemical bond.

Intra-cell edges, loop edges, and extension edges are preferably created by, for example, Voronoi tessellation between nodes.

Voronoi tessellation is a technique of dividing the nearest neighbor region of each node by drawing a perpendicular bisector on a straight line connecting adjacent nodes.

Here, a Voronoi diagram and Voronoi tessellation will be briefly described.

A Voronoi diagram is a diagram in which, with a plurality of points (generators) arranged at optional positions in a certain metric space, a region is divided on the basis of to which generator other points in the same metric space are closest. Furthermore, this division of the region is called Voronoi tessellation. A division pattern is determined only on the basis of the positions of the generators.

In a case where a unit cell of a crystalline material is schematically represented including chemical bonds, the chemical bonds in the unit cell of the crystalline material are usually not uniquely determined. However, creating edges (intra-cell edges, loop edges, and extension edges) by Voronoi tessellation makes it possible to uniquely determine edges (intra-cell edges, loop edges, and extension edges). As a result, it is possible to uniquely determine edges for a plurality of crystalline materials, and this improves an accuracy of analysis of crystalline materials.

Furthermore, intra-cell edges, loop edges, and extension edges may be created, for example, on the basis of a standard ionic radius of an atom. For example, it is possible to apply a rule in which an edge between nodes is created if $d \leq a(r1 + r2) + b$ is satisfied, where d is a distance between any two nodes, and r1 and r2 are the ionic radii of the nodes. Here, a is a constant equal to or larger than 0, and b is a constant. The constants are preferably $1.0 \leq a \leq 1.2$ and $0 \text{ Å} \leq b \leq 0.4 \text{ Å}$, but are not limited to these values.

As a method for creating intra-cell edges, loop edges, and extension edges, it is possible to freely select one or both of the two methods described above, the Voronoi tessellation method and the method using the standard ionic radius. In a case where both are selected, for example, among intra-cell edges, loop edges, and extension edges that may be determined by Voronoi tessellation, only those that are regarded as intra-cell edges, loop edges, and extension edges in the method using the standard ionic radius of the atom may be created as intra-cell edges, loop edges, and extension edges.

The crystalline materials are not particularly limited and can be appropriately selected depending on a purpose. Examples thereof include organic compounds, inorganic compounds, proteins, polymers, and the like.

The graph replication unit replicates a graph.

In the graph replication process, a graph is replicated.

A method of replicating a graph is not particularly limited and can be appropriately selected depending on a purpose.

The analysis unit analyzes a crystalline material by using a graph.

In the analysis process, a crystalline material is analyzed by using a graph.

Examples of analysis of a crystalline material include analysis of similarity in crystalline structure between a plurality of crystalline materials, prediction of characteristics of a crystalline material, and the like.

A method of analyzing similarity in crystalline structure between a plurality of crystalline materials is not particularly limited and can be appropriately selected depending on a purpose, as long as similarity between a crystalline structure of one crystalline material and a crystalline structure of another crystalline material is analyzed by using a graph according to the technique of the present application. Examples thereof include a method of calculating a score for the degree of similarity and determining that the larger the score, the higher the similarity, and the like.

The method of calculating a score for the degree of similarity is not particularly limited and can be appropriately selected depending on a purpose. Examples thereof include the following methods (1), (2), and the like.

(1) Fingerprint method
(2) A technique of analyzing similarity between compounds by searching for a substructure common to compounds by solving, with an annealing machine or the like, a maximum independent set problem in a conflict graph represented by an Ising model equation (e.g., see the following Non-Patent Document X)

Non-Patent Document X: Maritza Hernandez, Arman Zaribafiyan, Maliheh Aramon, Mohammad Naghibi "A Novel Graph-based Approach for Determining Molecular Similarity". ArXiv: 1601.06693 (https://arxiv.org/pdf/1601.06693.pdf)

In the fingerprint method, for example, whether the substructure of the query compound is included in the compound to be compared is represented by 0 or 1, and the degree of similarity is evaluated.

The maximum independent set problem is a problem in which, in a given graph G (V, E), among vertex sets $V' \subseteq V$, a set having no lines (edges) between vertices in V' (independent set) and having the largest size is obtained in graph theory.

Note that the method of calculating a score for the degree of similarity is not limited to these methods. For example, a score for the degree of similarity may be calculated on the basis of a cosine similarity, a correlation coefficient, a correlation function, an edit distance (also referred to as a Levenshtein distance), or the like related to calculation of the degree of similarity.

A method of predicting characteristics of a crystalline material is not particularly limited and can be appropriately selected depending on a purpose as long as it is a method of predicting characteristics of a crystalline material by using a graph according to the technique of the present application. Examples thereof include prediction of characteristics by using a learning model, and the like.

Examples of the prediction of characteristics by using a learning model include a method of creating a learning model by machine learning and predicting characteristics of a crystalline material by using the learning model, and the like.

Examples of machine learning include supervised learning, unsupervised learning, and the like.

The learning method in a case where a learning model is created by machine learning is not particularly limited and can be appropriately selected depending on a purpose.

Training data in machine learning includes, for example, a graph according to the technique of the present application and data on the characteristics of the crystalline material corresponding to the graph.

The characteristics are not particularly limited and can be appropriately selected depending on a purpose, and may be chemical characteristics or physical characteristics. Specific examples of the characteristics include electrical conductivity, ionic conductivity, discharge potential, relative permittivity, thermal conductivity, specific heat, and the like.

In a case of supervised learning, for example, learning is performed using a training data set including a graph of a certain crystalline material and characteristics of the crystalline material as a label.

Furthermore, in order to predict characteristics, a graph (graph structure data) according to the technique of the present application may be processed and used. For example, a graph may be converted into a tensor so that graph structure data may be learned with high accuracy by using machine learning (e.g., deep learning) technique.

Here, the tensor means data expressed as a multidimensional array in which concepts such as matrices and vectors have been commonalized.

The crystalline material analysis program disclosed in the present application may be recorded in a recording medium such as an internal hard disk or an external hard disk, or may be recorded in a recording medium such as a compact disc read only memory (CD-ROM), a digital versatile disk read only memory (DVD-ROM), a magneto-optical (MO) disk, or a universal serial bus (USB) flash drive.

Moreover, in a case where the crystalline material analysis program disclosed in the present application is recorded in a recording medium as described above, the program may be directly used, or may be installed on a hard disk and then used through a recording medium reader included in a computer system, depending on the situation. Furthermore, the crystalline material analysis program disclosed in the present application may be recorded in an external storage area (another computer or the like) accessible from the computer system through an information communication network. In this case, the crystalline material analysis program of the present application, which is recorded in an external storage area, may be used directly, or may be installed on a hard disk and then used from the external storage area through the information communication network, depending on the situation.

Note that the crystalline material analysis program of the present application may be divided for each of any pieces of processing, and then recorded in a plurality of recording media. In any of the aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein.

A recording medium disclosed in the present application is obtained by recording the crystalline material analysis program of the present application.

The recording medium is computer-readable.

The recording medium is not particularly limited and can be appropriately selected depending on a purpose. Examples of the recording medium include an internal hard disk, an external hard disk, a CD-ROM, a DVD-ROM, an MO disk, a USB flash drive, and the like.

Furthermore, the recording medium may include a plurality of recording media in which the crystalline material analysis program disclosed in the present application is recorded after being divided for each of any pieces of processing.

The recording medium may be transitory or non-transitory.

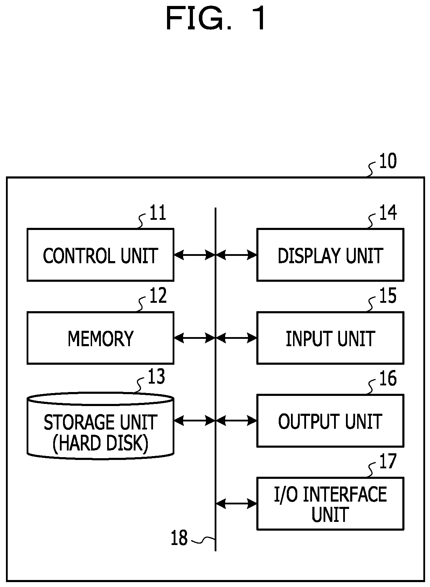

FIG. 1 illustrates an exemplary hardware configuration of the crystalline material analysis device of the present application.

In a crystalline material analysis device 10, for example, a control unit 11, a memory 12, a storage unit 13, a display unit 14, an input unit 15, an output unit 16, and an input/output (I/O) interface unit 17 are connected to each other via a system bus 18.

The control unit 11 performs arithmetic operations (for example, four arithmetic operations, comparison operations, and arithmetic operations for simulated annealing), hardware and software operation control, and the like.

The control unit 11 is not particularly limited and can be appropriately selected depending on a purpose; for example, the control unit 11 may be a CPU.

The graph creation unit, the graph replication unit, and the analysis unit of the crystalline material analysis device disclosed in the present application can be achieved by the control unit 11, for example.

The memory 12 is a memory such as a random access memory (RAM) or a read only memory (ROM). The RAM stores an operating system (OS), an application program, and the like read from the ROM and the storage unit 13, and functions as a main memory and a work area of the control unit 11.

The storage unit 13 is a device that stores various kinds of programs and data, and may be a hard disk, for example. The storage unit 13 stores a program to be executed by the control unit 11, data to be used in executing the program, an OS, and the like. For example, the graph database is stored in the storage unit 13.

Furthermore, the crystalline material analysis program disclosed in the present application is stored in, for example, the storage unit 13, is loaded into the RAM (main memory) of the memory 12, and is executed by the control unit 11.

The display unit 14 is a display device, and may be a display device such as a cathode ray tube (CRT) monitor or a liquid crystal panel, for example.

The input unit 15 is a device for inputting various kinds of data, and may be a keyboard, a pointing device (e.g., a mouse or the like), or the like, for example.

The output unit 16 is a device for outputting various kinds of data, and may be a printer or the like, for example.

The I/O interface unit 17 is an interface for connecting various external devices. The I/O interface unit 17 enables input and output of data on, for example, a CD-ROM, a DVD-ROM, an MO disk, a USB flash drive, or the like.

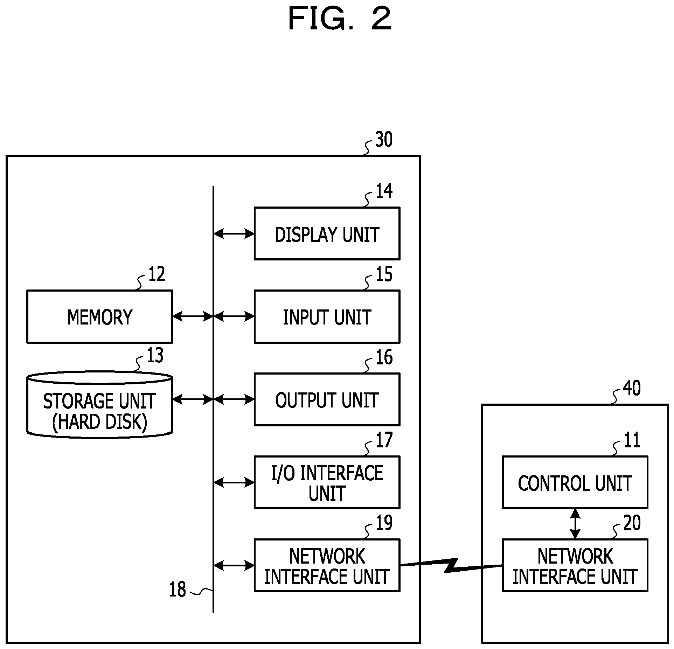

FIG. 2 illustrates another exemplary hardware configuration of the crystalline material analysis device disclosed in the present application.

The crystalline material analysis device illustrated in FIG. 2 is an example of a case where the crystalline material analysis device of a cloud type is employed, and the control unit 11 is independent of the storage unit 13 and the like. In the example illustrated in FIG. 2, a computer 30 that includes the storage unit 13 and the like is connected to a computer 40 that includes the control unit 11 via network interface units 19 and 20.

The network interface units 19 and 20 are hardware that performs communication using the Internet.

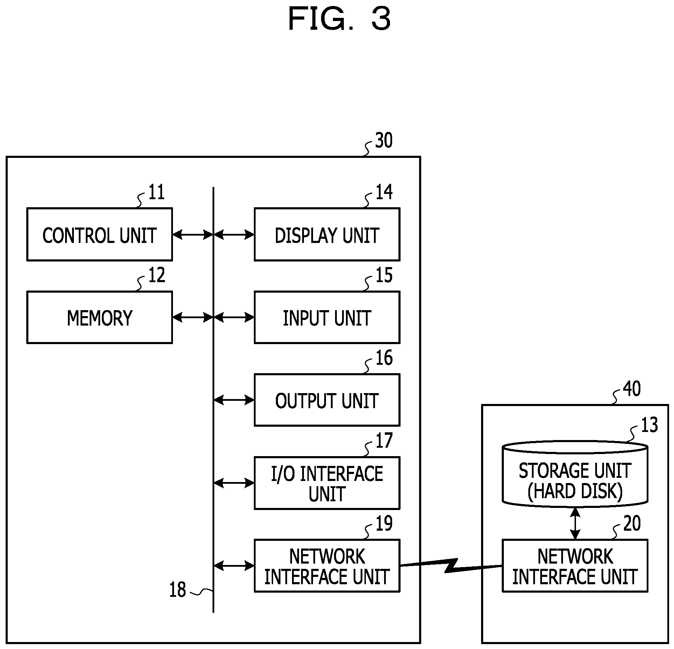

FIG. 3 illustrates another exemplary hardware configuration of the crystalline material analysis device disclosed in the present application.

The crystalline material analysis device illustrated in FIG. 3 is an example of a case where the crystalline material analysis device of a cloud type is employed, and the storage unit 13 is independent of the control unit 11 and the like. In the example illustrated in FIG. 3, the computer 30 that includes the control unit 11 and the like is connected to the computer 40 that includes the storage unit 13 via the network interface units 19 and 20.

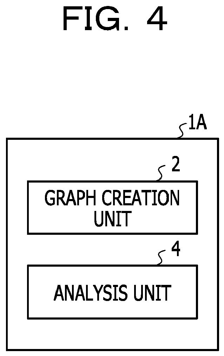

FIG. 4 illustrates an exemplary functional configuration as an embodiment of the crystalline material analysis device disclosed in the present application.

A crystalline material analysis device 1A illustrated in FIG. 4 includes a graph creation unit 2 and an analysis unit 4.

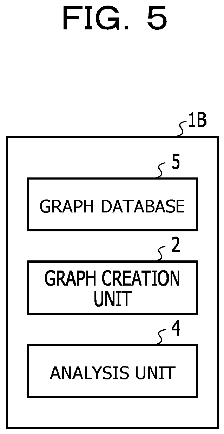

FIG. 5 illustrates an exemplary functional configuration as another embodiment of the crystalline material analysis device disclosed in the present application.

A crystalline material analysis device 1B illustrated in FIG. 5 includes the graph creation unit 2, the analysis unit 4, and a graph database 5.

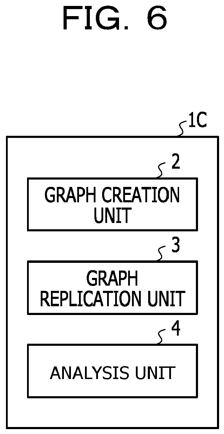

FIG. 6 illustrates an exemplary functional configuration as an embodiment of the crystalline material analysis device disclosed in the present application.

A crystalline material analysis device 1C illustrated in FIG. 6 includes the graph creation unit 2, a graph replication unit 3, and the analysis unit 4.

(First embodiment)

A crystalline material analysis device according to a first embodiment of the disclosed technique includes a graph creation unit and an analysis unit.

A crystalline material analysis method according to the first embodiment of the disclosed technique includes a computer performing a graph creation process and an analysis process.

A crystalline material program according to the first embodiment of the disclosed technique includes causing a computer to perform graph creation and analysis.

The graph creation unit creates a graph having an intra-cell node, an intra-cell edge, and a loop edge (hereinafter sometimes referred to as a "loop graph").

In the graph creation process, a loop graph is created.

The analysis unit analyzes a crystalline material by using a graph.

In the analysis process, a crystalline material is analyzed by using a graph.

For example, in the graph creation, a first loop graph of a unit cell of a first crystalline material as the loop graph and a second loop graph of a unit cell of a second crystalline material as the loop graph are created.

For example, in the analysis, similarity between the first crystalline material and the second crystalline material is analyzed by using the first loop graph and the second loop graph.

The analysis of similarity involves, for example, solving, with an annealing machine, a maximum independent set problem in a conflict graph represented by an Ising model equation.

The conflict graph is created on the basis of a rule that an edge is created between two nodes when the nodes are compared and are not identical to each other, and no edge is created between two nodes when the nodes are compared and are identical to each other, in which each of the nodes is a combination of one of node atoms constituting a unit cell of the first crystalline material expressed as a graph and one of node atoms constituting a unit cell of the second crystalline material expressed as a graph.

A maximum independent set in the conflict graph may be searched for by, for example, using a Hamiltonian in which minimizing means searching for the maximum independent set. More specifically, for example, the search may be performed by using a Hamiltonian (H) indicated by the following Formula (A).
[Math. 1]

$$H = -\alpha \sum_{i=0}^{n-1} b_i x_i + \beta \sum_{i,j=0}^{n-1} w_{ij} x_i x_j$$

... Formula (A)

Here, in the above Formula (A), n denotes the number of nodes in the conflict graph, and $b_i$ denotes a numerical value that represents a bias for an i-th node.

Moreover, $w_{ij}$ has a positive non-zero number when there is an edge between the i-th node and a j-th node, and has zero when there is no edge between the i-th node and the j-th node.

Furthermore, $x_i$ denotes a binary variable that represents that the i-th node has 0 or 1, and $x_j$ denotes a binary variable that represents that the j-th node has 0 or 1.

Note that $\alpha$ and $\beta$ denote positive numbers.

The annealing machine is not particularly limited and can be appropriately selected depending on a purpose, as long as the annealing machine is a computer that employs an annealing method of searching for a ground state for an energy function represented by an Ising model. Examples of the annealing machine include a quantum annealing machine, a semiconductor annealing machine using a semiconductor technology, a machine that performs simulated annealing executed by software using a CPU or a GPU, and the like. Furthermore, for example, Digital Annealer (registered trademark) may be used as the annealing machine.

Here, benefits of the first embodiment will be described.

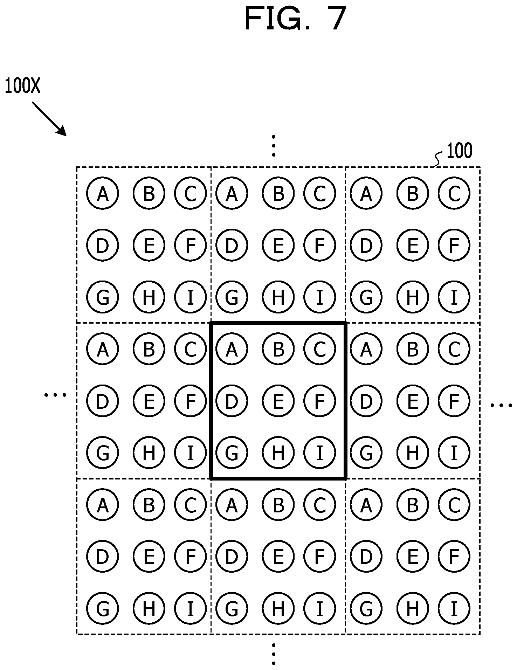

FIG. 7 is a schematic diagram of a crystalline structure of a crystalline material. The crystalline material in FIG. 7 has nine distinct types of atoms (atom A to atom I) in a unit cell.

A crystalline structure is constituted by infinite repetition of a unit cell. It is therefore usually unable to represent a crystalline structure by a graph. Even in a case where the graph is limited to a certain size, an extraordinarily large number of nodes and edges are used.

On the other hand, even in a case where a unit cell is represented by a graph, it is unable to represent only one crystalline structure with the graph, and the graph may result in including a plurality of crystalline structures in some cases. An example of this will be described.

In FIG. 7, a crystalline structure 100X of a crystalline material has a unit cell 100 including atom A to atom I as a repeating unit. Note that, in FIG. 7, dotted lines at the top, bottom, left, and right mean that the unit cell 100 is repeated.

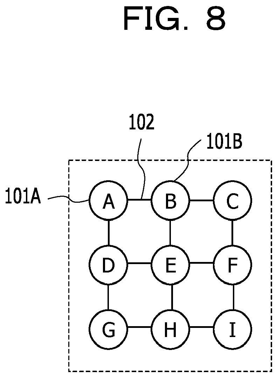

When the unit cell 100 of the crystalline structure 100X in FIG. 7 is represented by a graph, the graph is usually represented as illustrated in FIG. 8.

FIG. 8 is an example of a graph of the unit cell 100. The graph in FIG. 8 includes a node 101A (intra-cell node) indicating atom A, a node 101B (intra-cell node) indicating atom B, and an edge 102 (intra-cell edge) indicating a chemical bond between atom A and atom B. Note that, although reference numerals are omitted, the graph in FIG. 8 further includes 7 intra-cell nodes and 11 intra-cell edges.

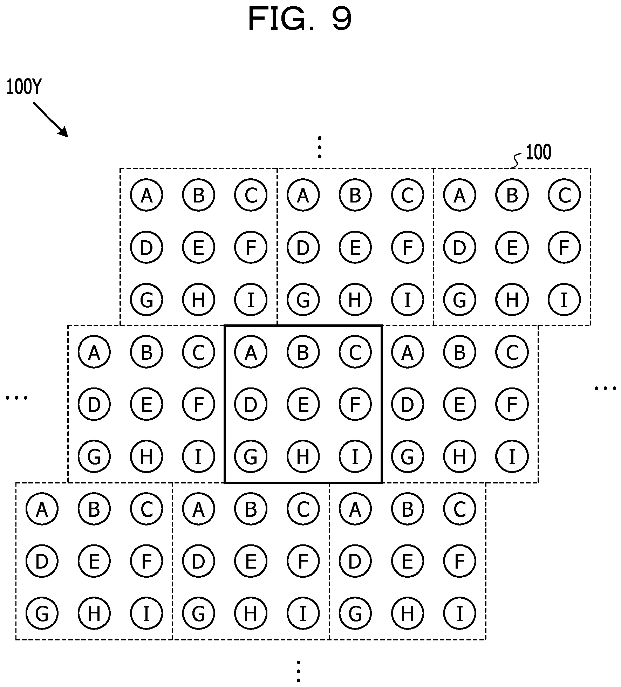

Here, examples of a crystalline structure having the unit cell 100 as a repeating unit include, in addition to the crystalline structure 100X in FIG. 7, a crystalline structure 100Y in FIG. 9, and the like. Note that, in FIG. 9, dotted lines at the top, bottom, left, and right mean that the unit cell 100 is repeated.

Then, in the same method as that used to represent the unit cell 100 of the crystalline structure 100X in FIG. 7 by the graph in FIG. 8, the unit cell of the crystalline structure 100Y in FIG. 9 is represented by the graph in FIG. 8.

Then, the graph in FIG. 8 is not a graph representing only one crystalline structure.

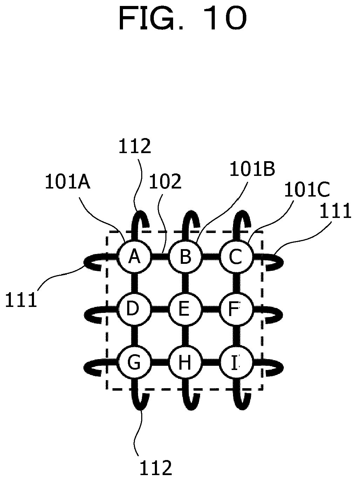

On the other hand, in a case of a graph created in the first embodiment according to the technique of the present application, a graph corresponding to the crystalline structure in FIG. 7 is a loop graph as illustrated in FIG. 10. FIG. 10 is a loop graph related to the unit cell 100 of the crystalline structure in FIG. 7.

The loop graph in FIG. 10 includes the node 101A (intra-cell node) indicating atom A in the unit cell 100, the node 101B (intra-cell node) indicating atom B in the unit cell 100, a node 101C (intra-cell node) indicating atom C in the unit cell 100, and also six intra-cell nodes without reference numerals.

Furthermore, the loop graph in FIG. 10 has the edge 102 (intra-cell edge) indicating a bonding relationship between atom A and atom B in the unit cell 100, and also 11 intra-cell edges without reference numerals.

Moreover, the loop graph in FIG. 10 has a total of six loop edges. Two of the loop edges are an edge 111 and an edge 112. Note that, in the loop graph in FIG. 10, the edge 111 at the left and the edge 111 at the right are the same loop edge. Furthermore, in the loop graph in FIG. 10, the edge 112 at the top and the edge 112 at the bottom are the same loop edge.

The edge 111 (loop edge) indicates a virtual chemical bond between atom A in the unit cell 100 and atom C in the unit cell 100 corresponding to atom C in an adjacent unit cell having a chemical bond with atom A in the unit cell 100. Note that the edge 111 also indicates a virtual chemical bond between atom C in the unit cell 100 and atom A in the unit cell 100 corresponding to atom A in an adjacent unit cell having a chemical bond with atom C in the unit cell 100.

The edge 112 (loop edge) indicates a virtual chemical bond between atom A in the unit cell 100 and atom G in the unit cell 100 corresponding to atom G in an adjacent unit cell having a chemical bond with atom A in the unit cell 100. Note that the edge 112 also indicates a virtual chemical bond between atom G in the unit cell 100 and atom A in the unit cell 100 corresponding to atom A in an adjacent unit cell having a chemical bond with atom G in the unit cell 100.

By providing loop edges in a graph in this way, it is possible to obtain a graph including information regarding a structure of repetition of a unit cell (information regarding how unit cells are arranged) without increasing the number of nodes in the graph.

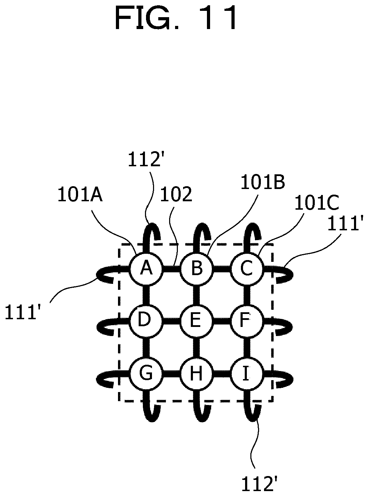

On the other hand, in a case of a graph created in the first embodiment according to the technique of the present application, a graph corresponding to the crystalline structure in FIG. 9 is a loop graph as illustrated in FIG. 11. FIG. 11 is a loop graph related to the unit cell 100 of the crystalline structure in FIG. 9.

Here, the loop graph in FIG. 11 has a total of six loop edges in the same manner as the loop graph in FIG. 10. Then, two of the loop edges are an edge 111' and an edge 112'. Note that, in the loop graph in FIG. 11, the edge 111' at the left and the edge 111' at the right are the same loop edge. Furthermore, in the loop graph in FIG. 11, the edge 112' at the top and the edge 112' at the bottom are the same loop edge.

The edge 111' (loop edge) indicates a virtual chemical bond between atom A in the unit cell 100 and atom C in the unit cell 100 corresponding to atom C in an adjacent unit cell having a chemical bond with atom A in the unit cell 100. Note that the edge 111' also indicates a virtual chemical bond between atom C in the unit cell 100 and atom A in the unit cell 100 corresponding to atom A in an adjacent unit cell having a chemical bond with atom C in the unit cell 100.

The edge 112' (loop edge) indicates a virtual chemical bond between atom A in the unit cell 100 and atom I in the unit cell 100 corresponding to atom I in an adjacent unit cell having a chemical bond with atom A in the unit cell 100. Note that the edge 112' also indicates a virtual chemical bond between atom I in the unit cell 100 and atom A in the unit cell 100 corresponding to atom A in an adjacent unit cell having a chemical bond with atom I in the unit cell 100.

Here, the edge 111 and the edge 111' are the same edge, but the edge 112 and the edge 112' are different edges. Due to the difference between the edge 112 and the edge 112', the loop graph in FIG. 10 and the loop graph in FIG. 11 are different graphs.

Summarizing the above, in a case where a unit cell is expressed as a graph as illustrated in FIG. 8, the graph is not a graph representing only one crystalline structure.

On the other hand, in the graphing technique of the first embodiment, two or more crystalline structures that are the same in unit cell but different in crystalline structure may be represented by different graphs.

In a case where it is possible to represent two or more crystalline structures that are different in crystalline structure by different graphs, it is possible to improve the analysis accuracy in analysis of crystalline materials (e.g., analysis of similarity between crystalline materials, prediction of characteristics of crystalline materials, and the like).

Furthermore, the number of nodes may affect the number of bits of a calculator, and the smaller the number of nodes, the better. On the other hand, the number of edges may affect the accuracy of analysis, and the larger the number of edges, the better. Here, as compared with a common graph that does not include loop edges, a loop graph allows for an increase in the number of edges without an increase in the number of nodes.

A loop graph is therefore expected to improve the analysis accuracy without an increase in the number of bits of a calculator.

The graphing technique of the first embodiment is therefore useful for analysis of a crystalline material using computer technology.

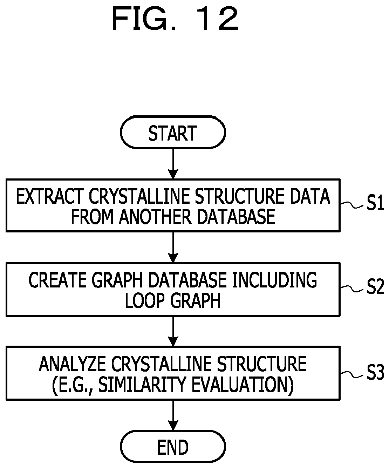

The crystalline material analysis method in the first embodiment will be briefly described with reference to a flowchart illustrated in FIG. 12.

First, crystalline structure data is extracted from another database (S1). The other database is not particularly limited and can be appropriately selected depending on a purpose. Examples thereof include an inorganic crystal structure database (ICSD) and the like. Note that the number of pieces and the types of crystalline structure data to be extracted are not particularly limited and can be appropriately selected depending on a purpose. For example, only crystalline structure data on chemicals having a specific element may be extracted.

Next, using the extracted crystalline structure data, a loop graph is created by graphing according to the technique of the present application, and a graph database including the loop graph is created (S2). The loop graph is created, for example, in accordance with flowcharts described later.

Next, the loop graph in the graph database is used to analyze a crystalline structure (S3). Examples of the analysis include similarity evaluation. The similarity evaluation is performed by, for example, solving, with the annealing machine or the like, a maximum independent set problem in a conflict graph represented by an Ising model equation. The method may be performed, for example, with reference to the following non-patent document.

Non-Patent Document: Maritza Hernandez, Arman Zaribafiyan, Maliheh Aramon, Mohammad Naghibi "A Novel Graph-based Approach for Determining Molecular Similarity". ArXiv: 1601.06693 (https://arxiv.org/pdf/1601.06693.pdf)

Thus, it is possible to evaluate similarity in crystalline structure, which is one of analyses of a crystalline structure.

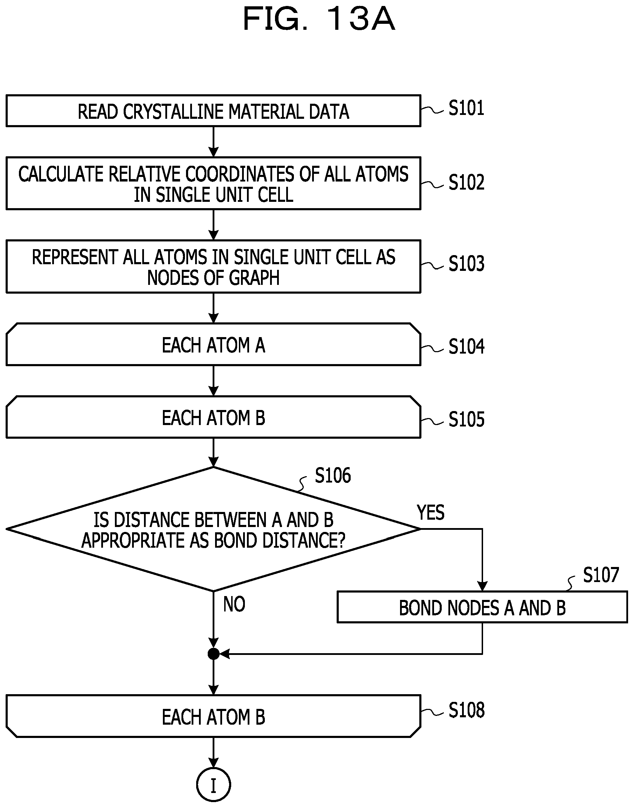

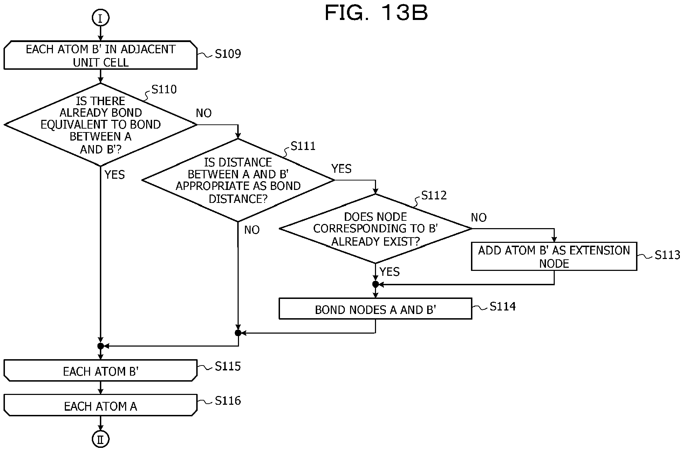

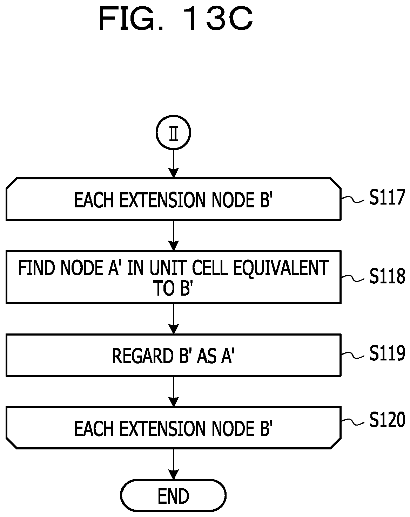

Here, an example of a loop graph creation method will be described using flowcharts. FIGs. 13A to 13C are flowcharts of an example of the loop graph creation method.

First, crystalline material data is read (S101).

Next, relative coordinates of all atoms in a single unit cell are calculated (S102). As a result, relative positional relationships of all the atoms are obtained.

Next, all the atoms in the single unit cell are represented as nodes of a graph (S103). These nodes are intra-cell nodes.

Next, for a combination of two atoms (A and B), that is, a certain atom (atom A) and another atom (atom B) other than the certain atom among all the atoms in the single unit cell, it is determined whether the distance between A and B is appropriate as a bond distance (S106) . Then, in a case where appropriate, node A and node B are bonded (S107). In other words, an edge is provided between node A and node B. This edge is an intra-cell edge. Thereafter, S106 is performed for all combinations of the certain atom (atom A) and yet another atom B (S105 and S108). If not appropriate, an edge is not provided between node A and node B, and S106 is performed for all combinations of the certain atom (atom A) and yet another atom B (S105 and S108).

Next, it is determined whether there is already a bond, which is equivalent to a bond between A and B', between the certain atom (atom A) in the single unit cell and any atom B' in an adjacent unit cell (S110). If there is no bond equivalent to the bond between A and B' yet, it is determined whether the distance between A and B' is appropriate as a bond distance (Sill). If the distance between A and B' is appropriate as a bond distance, it is determined whether a node corresponding to B' already exists (S112). Thereafter, if there is no node corresponding to B' yet, atom B' is added as an extension node (S113) . Thereafter, node A and node B' are bonded (S114). In other words, an edge is provided between node A and node B'. As a result of the determination in S112, if a node corresponding to B' already exists, node A and node B' are bonded (S114). If a result of the determination in S110 indicates that the distance between A and B' is not appropriate as a bond distance, if a result of the determination in S111 indicates that a node corresponding to B' already exists, and after S114 has been performed, the processes between S109 and S115 are performed for all combinations of the certain atom (atom A) and yet another atom B'.

The processes of S105 to S115 are performed for all atoms corresponding to the certain atom A in the single unit cell (S104 and S116).

Next, node A' in the unit cell equivalent to extension node B' is found (S118). Then, extension node B' is regarded as node A' (S119). Then, an edge between node A and node A' becomes a loop edge. These processes are performed on all of the extension nodes (S117 and S120).

As a result, a loop graph is obtained.

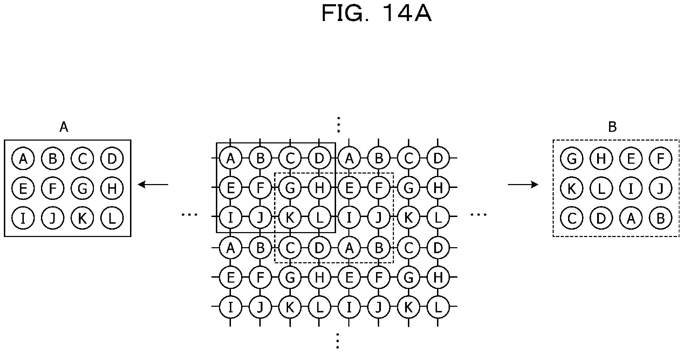

Here, an example of a comparison between the degree of similarity in a case of a conventional graph and the degree of similarity in a case of a loop graph will be described with reference to FIGs. 14A to 14C.

A crystalline structure constituted by 12 distinct types of atoms A to L, as illustrated in FIG. 14A, is used. A case is considered where two types of unit cells, unit cell A and unit cell B illustrated in FIG. 14A, are recognized from this crystalline structure.

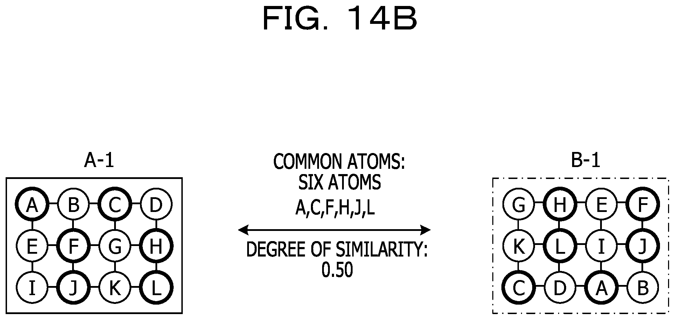

In a conventional graphing technique, a graph created for unit cell A is graph A-1 illustrated in FIG. 14B, and a graph created for unit cell B is graph B-1 illustrated in FIG. 14B. Graph A-1 and graph B-1 are represented by an Ising model equation and solved by the annealing machine as a maximum independent set problem in a conflict graph. Then, six atoms (A, C, F, H, J, and L) are found as common atoms, and the degree of similarity obtained by Formula (1-1) described later is 0.50.

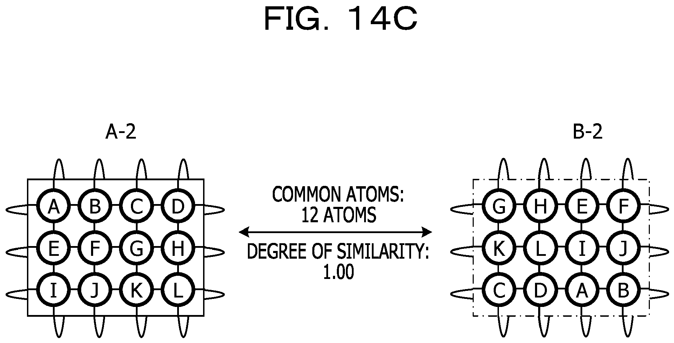

On the other hand, when a loop graph is created by the graphing technique of the first embodiment, a graph created for unit cell A is graph A-2 illustrated in FIG. 14C, and a graph created for unit cell B is graph B-2 illustrated in FIG. 14C. Graph A-2 and graph B-2 are represented by an Ising model equation and solved by the annealing machine as a maximum independent set problem in a conflict graph. Then, 12 atoms are found as common atoms, and the degree of similarity obtained by Formula (1-1) described later is 1.00.

Unit cell A and unit cell B have the same crystalline structure. Therefore, the degree of similarity between the graph created for unit cell A and the graph created for unit cell B being 1.00 means that the calculation result is more accurate.

In this way, a loop graph may be used to improve the accuracy of analysis (e.g., the degree of similarity).

<Second embodiment>

A crystalline material analysis device according to a second embodiment of the disclosed technique includes a graph creation unit, a graph replication unit, and an analysis unit.

A crystalline material analysis method according to the second embodiment of the disclosed technique includes a computer performing a graph creation process, a graph replication process, and an analysis process.

A crystalline material program according to the second embodiment of the disclosed technique includes causing a computer to perform graph creation, graph replication, and analysis.

The graph creation unit creates a graph having an intra-cell node and an intra-cell edge.

In the graph creation process, a graph having an intra-cell node and an intra-cell edge is created.

In the graph creation, a graph having an intra-cell node, an intra-cell edge, an extension node, and an extension edge (hereinafter sometimes referred to as an "extension graph") may be created. The extension graph may have an edge between extension nodes.

In the graph creation, a loop graph may be created.

Note that a graph having only intra-cell nodes as nodes and only intra-cell edges as edges may be hereinafter sometimes referred to as a "simple graph".

The graph replication unit generates a replicated graph by replicating a graph.

In the graph replication process, a replicated graph is generated by replicating a graph.

The analysis unit analyzes a crystalline material by using a replicated graph.

In the analysis process, a crystalline material is analyzed by using a replicated graph.

For example, in the graph creation, a first graph of a unit cell of a first crystalline material as a graph and a second graph of a unit cell of a second crystalline material as a graph are created.

For example, in the graph replication, at least one of a first graph or a second graph is replicated.

The replication of at least one of the first graph or the second graph is performed in such a way as to, for example, minimize an absolute value of a difference between the total number of nodes in a plurality of the first graphs after the replication and the total number of nodes in a plurality of the second graphs after the replication.

For example, in the analysis, similarity between the first crystalline material and the second crystalline material is analyzed by using any one of the first graph or a first integrated graph and any one of the second graph or a second integrated graph.

The first integrated graph is obtained by integrating, with one another, a plurality of first replicated graphs generated by replicating the first graph.

The second integrated graph is obtained by integrating, with one another, a plurality of second replicated graphs generated by replicating the second graph.

Here, a graph obtained by integrating replicated graphs (hereinafter sometimes referred to as an "integrated graph") may be, for example, a graph in which a plurality of graphs obtained by replication is simply put together. In this case, there are no edges between the plurality of graphs.

Furthermore, a graph obtained by integrating replicated graphs may be, for example, a graph obtained by combining, by edges (hereinafter sometimes referred to as "edges between graphs"), a plurality of graphs obtained by replication. In this case, the edges between graphs are preferably added between the plurality of graphs so that the integrated graph matches the structure of the crystalline material.

The analysis of similarity involves, for example, solving, with an annealing machine, a maximum independent set problem in a conflict graph represented by an Ising model equation.

Here, an example of the second embodiment will be briefly described below.

A case where each of the graphs of the two crystalline materials is replicated is described below, but only one of the graphs may be replicated.

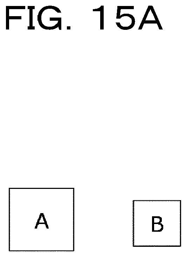

A case is assumed in which two unit cells A and B illustrated in FIG. 15A are analyzed. As for the analysis, calculation of the degree of similarity is taken as an example. Here, a simple graph of unit cell A is referred to as graph A. A simple graph of unit cell B is referred to as graph B. In a case where the number of nodes in graph A and the number of nodes in graph B are different, the difference causes a decrease in the degree of similarity. The reason will be described later.

Thus, in order to reduce a difference between the number of nodes in graph A and the number of nodes in graph B, at least one of graph A or graph B is replicated. The replication is performed, for example, in such a way as to minimize an absolute value of a difference between the total number of nodes in graph A after the replication and the total number of nodes in graph B after the replication.

For example, graph A is replicated four times and graph B is replicated nine times.

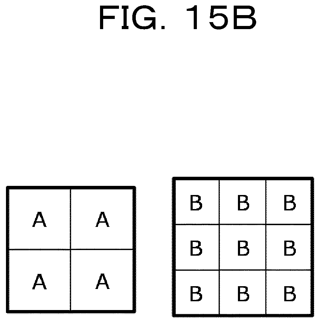

As for an integrated graph obtained by integrating replicated graphs, for example, graphs A may be combined, or graphs B may be combined as illustrated in FIG. 15B. Combining here means providing edges between the graphs (between graphs A or between graphs B).

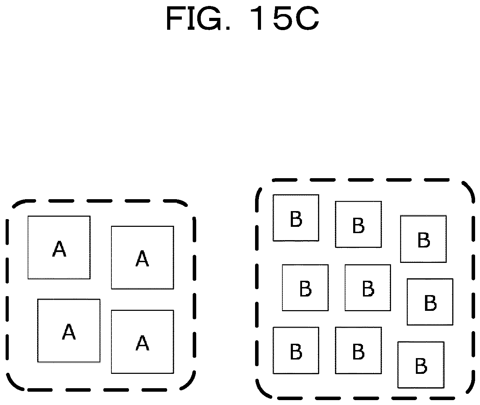

On the other hand, in an integrated graph, graphs A may not be combined, or graphs B may not be combined (FIG. 15C).

Then, analysis is performed using an integrated graph obtained by replicating a graph in this way.

Note that, for example, integrated graphs of the second embodiment may be classified into the following combinations, for example, depending on a structure of the graph.

- 2-1: There are bonds (edges between graphs) between the graphs in the integrated graph (FIG. 15B).

-- 2-1-1: The integrated graph is a simple graph.
-- 2-1-2: The integrated graph is an extension graph.
-- 2-1-3: The integrated graph is a loop graph.

- 2-2: There are no bonds (edges between graphs) between the graphs in the integrated graph (FIG. 15C).

-- 2-2-1: Each graph in the integrated graph is a simple graph.
-- 2-2-2: Each graph in the integrated graph is an extension graph.
-- 2-2-3: Each graph in the integrated graph is a loop graph.

Here, benefits of the second embodiment will be described.

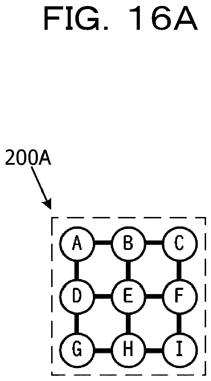

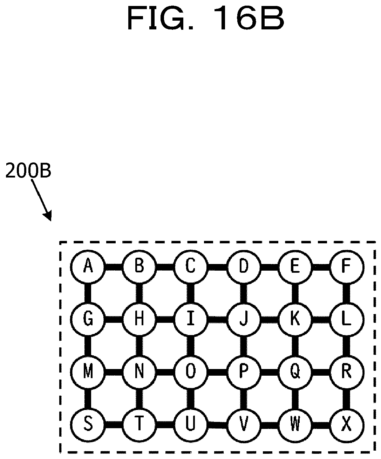

FIGs. 16A and 16B are schematic diagrams of a unit cell.

A unit cell 200A in FIG. 16A has nine distinct types of atoms (atom A to atom I).

A unit cell 200B in FIG. 16B includes 24 distinct types of atoms (atom A to atom X).

Here, the degree of similarity between the unit cell 200A and the unit cell 200B is calculated by using a simple graph constituted by the unit cell 200A in FIG. 16A and a simple graph constituted by the unit cell 200B in FIG. 16B. In the calculation of the degree of similarity by using a maximum independent set, the degree of similarity is commonly obtained by, for example, the following Formula (1).

[Math. 2]

$$S(G_A, G_B) = \delta \max\left\{\frac{|V_C^A|}{|V_A|}, \frac{|V_C^B|}{|V_B|}\right\} + (1 - \delta) \min\left\{\frac{|V_C^A|}{|V_A|}, \frac{|V_C^B|}{|V_B|}\right\} \quad \text{.. Formula (1)}$$

Here, in the above Formula (1), S ($G_A$, $G_B$) represents the degree of similarity between unit cell A of the first crystalline material expressed as a graph and unit cell B of the second crystalline material expressed as a graph, is represented as 0 to 1, and means that the closer to 1, the higher the degree of similarity.

Furthermore, $V_A$ represents the total number of node atoms in unit cell A expressed as a graph, and $V_c^A$ represents the number of node atoms included in a maximum independent set in a conflict graph, among node atoms in unit cell A expressed as a graph. Note that the node atoms mean atoms at vertices of a molecule expressed as a graph.

Moreover, $V_B$ represents the total number of node atoms in unit cell B expressed as a graph, and $V_c^B$ represents the number of node atoms included in the maximum independent set in the conflict graph among node atoms in unit cell B expressed as a graph.

$\delta$ denotes a number from 0 to 1.

Furthermore, in the above Formula (1), max{A, B} means to select A or B, whichever has a larger value, and min{A, B} means to select A or B, whichever has a smaller value.

Here, the conflict graph is understood as a graph that is created on the basis of a rule that an edge is created between two nodes when the nodes are compared and are not identical to each other, and no edge is created between two nodes when the nodes are compared and are identical to each other, in which each of the nodes is a combination of one of node atoms constituting unit cell A of the first crystalline material expressed as a graph and one of node atoms constituting unit cell B of the second crystalline material expressed as a graph.

The above Formula (1) may be simplified to the following Formula (1-1). $\delta$ is 0.5.

[Math. 3]

$$\text{(Degree of similarity)} = \frac{1}{2}\left(\frac{AB_{common}}{A_{all}} + \frac{AB_{common}}{B_{all}}\right) \quad \text{... Formula (1-1)}$$

Here, $AB_{common}$ represents the number of substructures common to unit cell A and unit cell B. $A_{all}$ represents the number of nodes in a graph of unit cell A, and $B_{all}$ represents the number of nodes in a graph of unit cell B.

In a case where all atoms in the unit cell 200A in FIG. 16A constitute substructure common to the atoms in the unit cell 200A and the atoms in the unit cell 200B in FIG. 16B, the degree of similarity is maximized. In that case, the numerical value of the degree of similarity obtained from Formula (1-1) is 1/2[(9/9) + (9/24)] = 0.675. This means that the difference between the numbers of atoms (the numbers of nodes) in the two unit cells reduces the degree of similarity.

Thus, in the second embodiment, at least one of graphs of unit cells of two crystalline materials is replicated in the graph replication. By replicating the graph so as to reduce the difference in the number of atoms (the number of nodes) between the two unit cells to be compared, it is possible to avoid a decrease in the degree of similarity caused by the difference in the number of atoms (the number of nodes). As a result, the accuracy of analysis (e.g., the degree of similarity) may be improved.

The graphing technique of the second embodiment is therefore useful for analysis of a crystalline material using computer technology.

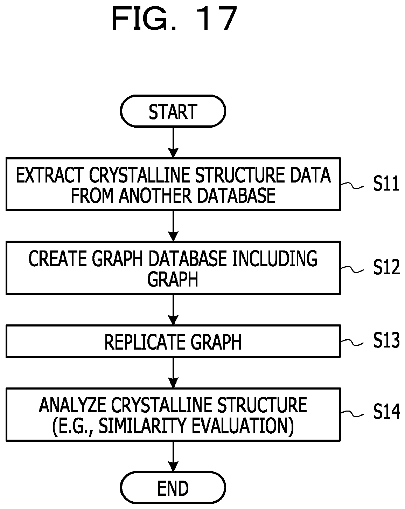

The crystalline material analysis method in the second embodiment will be briefly described with reference to a flowchart illustrated in FIG. 17.

First, crystalline structure data is extracted from another database (S11). The other database is not particularly limited and can be appropriately selected depending on a purpose. Examples thereof include an inorganic crystal structure database (ICSD) and the like. Note that the number of pieces and the types of crystalline structure data to be extracted are not particularly limited and can be appropriately selected depending on a purpose. For example, only crystalline structure data on chemicals having a specific element may be extracted.

Next, using the extracted crystalline structure data, a graph is created by graphing according to the technique of the present application, and a graph database including the graph is created (S12).

Next, a replicated graph is generated by replicating the created graph (S13) .

Next, a crystalline structure is analyzed by using the replicated graph (S14). Examples of the analysis include similarity evaluation. The similarity evaluation is performed by, for example, solving, with the annealing machine or the like, a maximum independent set problem in a conflict graph represented by an Ising model equation.

Thus, it is possible to evaluate similarity in crystalline structure, which is one of analyses of a crystalline structure.

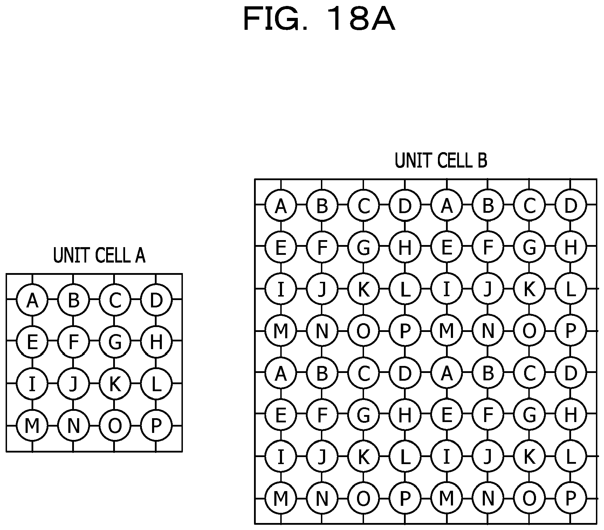

Here, a working example of the second embodiment will be described with reference to FIGs. 18A to 18C, FIGs. 19A to 19C, FIGs. 20A to 20C, and FIGs. 21A to 21C.

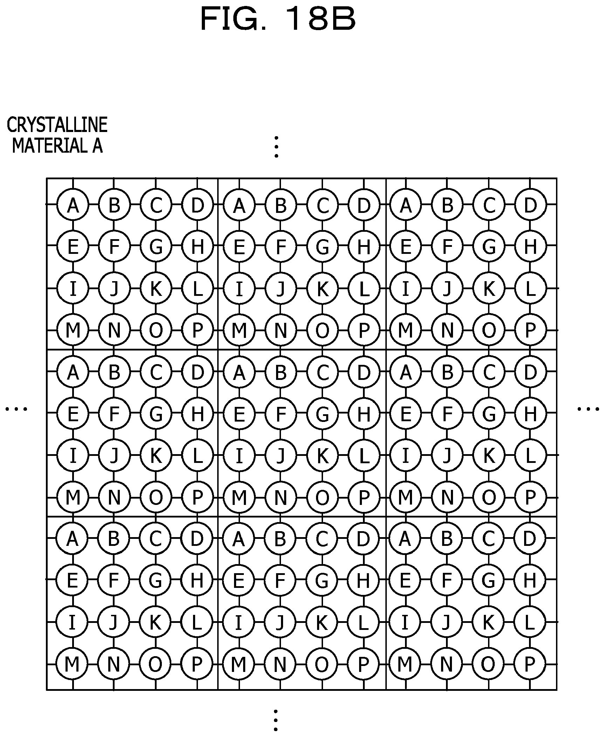

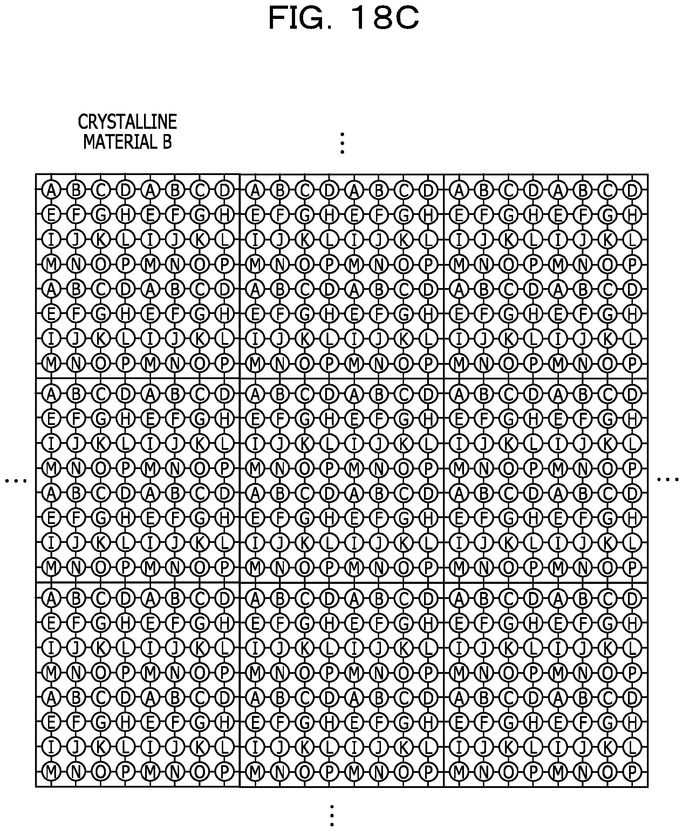

In this working example, the degree of similarity between crystalline material A represented by unit cell A and crystalline material B represented by unit cell B illustrated in FIG. 18A is obtained. Crystalline material A represented by unit cell A has a structure in which unit cell A is repeated as illustrated in FIG. 18B. Crystalline material B represented by unit cell B has a structure in which unit cell B is repeated as illustrated in FIG. 18C. Unit cell A and unit cell B are constituted by 16 distinct types of atoms A to P.

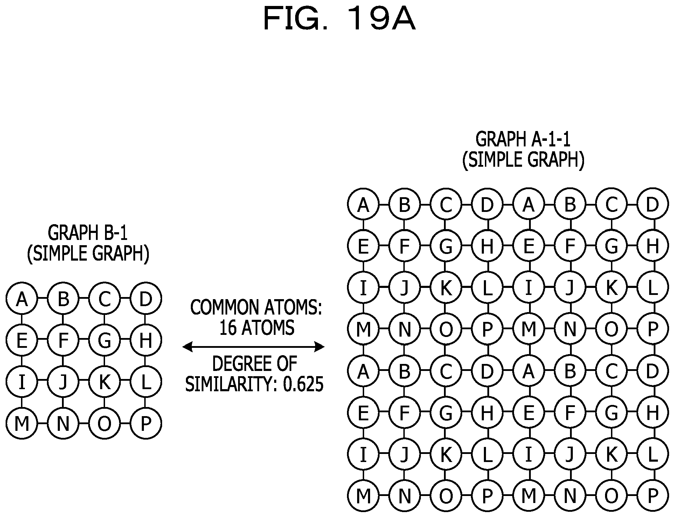

A simple graph created from unit cell A illustrated in FIG. 18A is graph A-1-1 illustrated in FIG. 19A.

A simple graph created from unit cell B illustrated in FIG. 18A is graph B-1 illustrated in FIG. 19A.

Graph A-1-2 and graph B-1 are represented by an Ising model equation and solved by the annealing machine as a maximum independent set problem in a conflict graph. Then, 16 atoms are found as common atoms, and the degree of similarity obtained by Formula (1-1) described previously is 0.625 (FIG. 19A). This example is a comparative example.

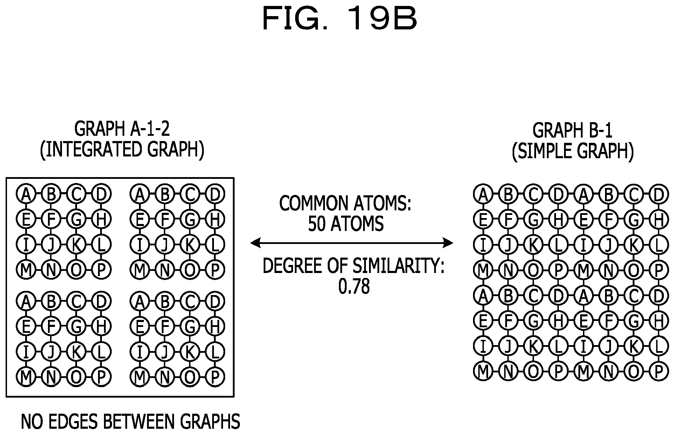

On the other hand, as an example of the second embodiment, an integrated graph (graph A-1-2, no edges between graphs) obtained by replicating graph A-1-1 (simple graph) four times is created (FIG. 19B). Then, graph A-1-2 and graph B-1 are represented by an Ising model equation and solved by the annealing machine as a maximum independent set problem in a conflict graph. Then, 50 atoms are found as common atoms, and the degree of similarity obtained by Formula (1-1) described previously is 0.78 (FIG. 19B).

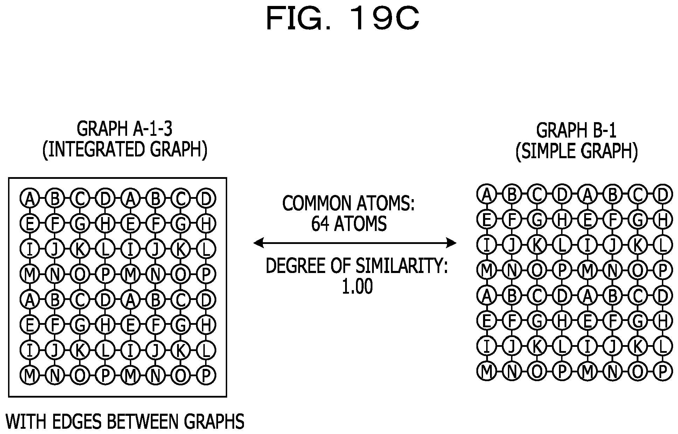

Furthermore, as an example of the second embodiment, an integrated graph (graph A-1-3, with edges between graphs) obtained by replicating graph A-1-1 (simple graph) four times is created (FIG. 19C). In this case, graph A-1-3 and graph B-1 are the same graph. Then, graph A-1-3 and graph B-1 are represented by an Ising model equation and solved by the annealing machine as a maximum independent set problem in a conflict graph. Then, 64 atoms are found as common atoms, and the degree of similarity obtained by Formula (1-1) described previously is 1.00 (FIG. 19C).

Next, a working example of a case where an extension graph is used will be described.

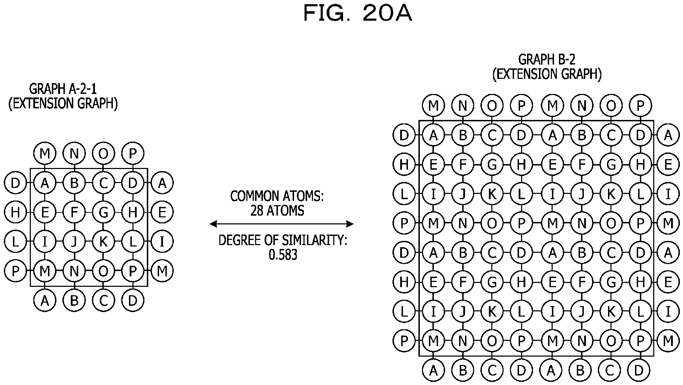

When an extension graph is created from unit cell A illustrated in FIG. 18A, graph A-2-1 illustrated in FIG. 20A is obtained.

On the other hand, when an extension graph is created from unit cell B illustrated in FIG. 18A, graph B-2 illustrated in FIG. 20A is obtained.

Then, graph A-2-1 and graph B-2 are represented by an Ising model equation and solved by the annealing machine as a maximum independent set problem in a conflict graph. Then, 28 atoms are found as common atoms, and the degree of similarity obtained by Formula (1-1) described previously is 0.583 (FIG. 20A). In this example, the graphs are not replicated, which makes this example different from the second embodiment.

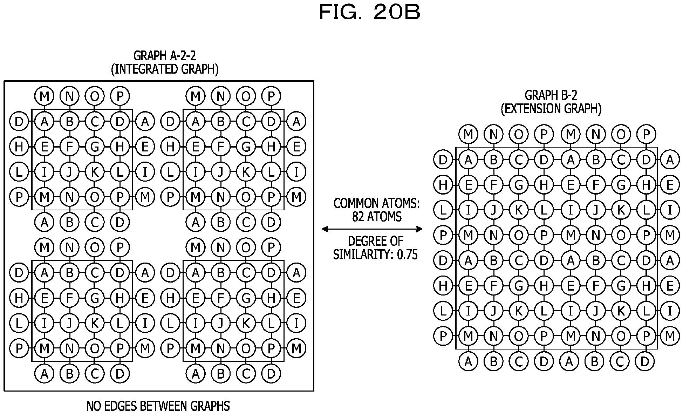

On the other hand, as an example of the second embodiment, an integrated graph (graph A-2-2, no edges between graphs) obtained by replicating graph A-2-1 (extension graph) four times is created (FIG. 20B). In this case, the integrated graph (graph A-2-2) may be obtained by replicating graph A-2-1 (extension graph) four times, or the integrated graph (graph A-2-2) may be obtained by replicating graph A-1-1 (simple graph) four times and then converting each of them into an extension graph. Then, graph A-2-2 and graph B-2 are represented by an Ising model equation and solved by the annealing machine as a maximum independent set problem in a conflict graph. Then, 82 atoms are found as common atoms, and the degree of similarity obtained by Formula (1-1) described previously is 0.75 (FIG. 20B).

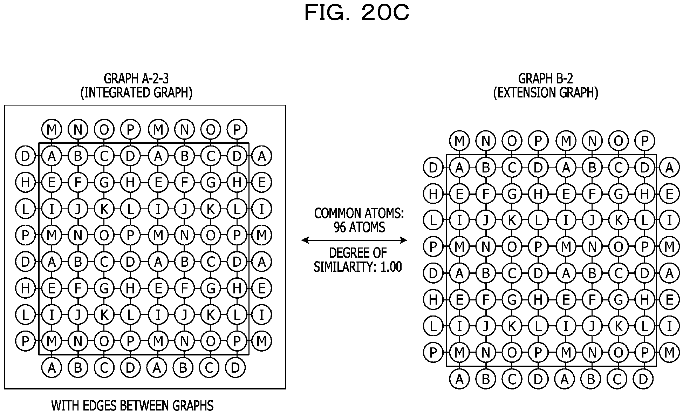

Furthermore, as an example of the second embodiment, an integrated graph (graph A-2-3, extension graph, with edges between graphs) obtained by replicating graph A-1-1 (simple graph) four times is created (FIG. 20C). In this case, the integrated graph (graph A-2-3) is obtained by, for example, replicating graph A-1-1 (simple graph) four times and then combining graphs A-1-1, and converting a graph obtained by the combination into an extension graph. In this case, graph A-2-3 and graph B-2 are the same graph. Then, graph A-2-3 and graph B-2 are represented by an Ising model equation and solved by the annealing machine as a maximum independent set problem in a conflict graph. Then, 96 atoms are found as common atoms, and the degree of similarity obtained by Formula (1-1) described previously is 1.00 (FIG. 20C).

Next, a working example of a case where a loop graph is used will be described.

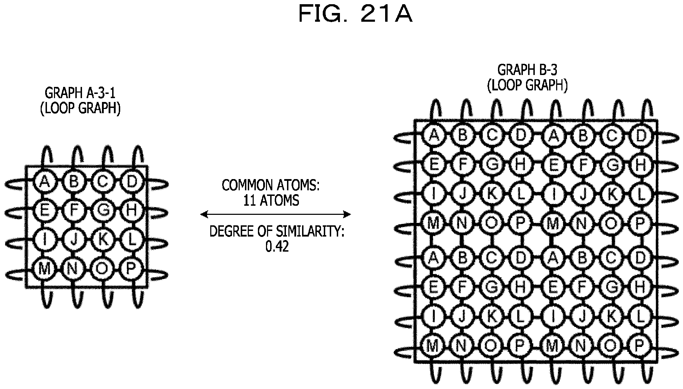

When a loop graph is created from unit cell A illustrated in FIG. 18A, graph A-3-1 illustrated in FIG. 21A is obtained.

On the other hand, when a loop graph is created from unit cell B illustrated in FIG. 18A, graph B-3 illustrated in FIG. 21A is obtained.

Then, graph A-3-1 and graph B-3 are represented by an Ising model equation and solved by the annealing machine as a maximum independent set problem in a conflict graph. Then, 11 atoms are found as common atoms, and the degree of similarity obtained by Formula (1-1) described previously is 0.42 (FIG. 21A). In this example, the graphs are not replicated, which makes this example different from the second embodiment.

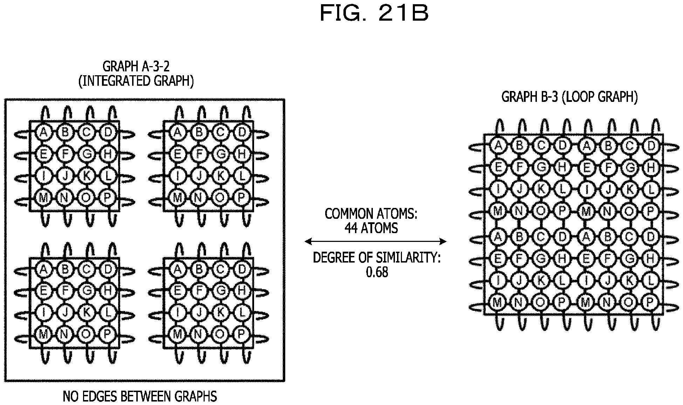

On the other hand, as an example of the second embodiment, an integrated graph (graph A-3-2, no edges between graphs) obtained by replicating graph A-3-1 (loop graph) four times is created. In this case, the integrated graph (graph A-3-2) may be obtained by replicating graph A-3-1 (loop graph) four times, or the integrated graph (graph A-3-2) may be obtained by replicating graph A-1-1 (simple graph) four times and then converting them into loop graphs. Then, graph A-3-2 and graph B-3 are represented by an Ising model equation and solved by the annealing machine as a maximum independent set problem in a conflict graph. Then, 44 atoms are found as common atoms, and the degree of similarity obtained by Formula (1-1) described previously is 0.68 (FIG. 21B).

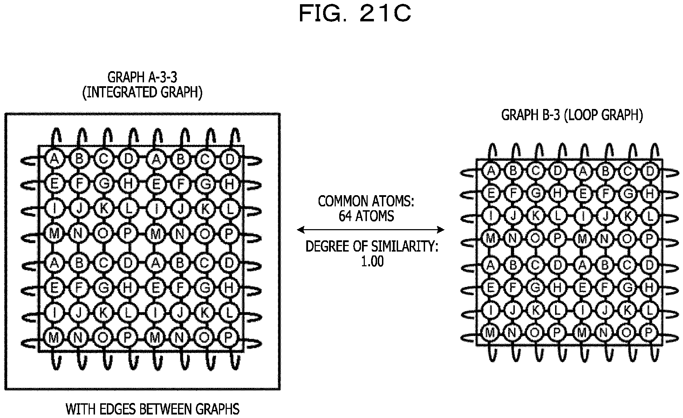

Furthermore, as an example of the second embodiment, an integrated graph (graph A-2-3, loop graph, with edges between graphs) obtained by replicating graph A-1-1 (simple graph) four times is created. In this case, an integrated graph (graph A-3-3) is obtained by, for example, replicating graph A-1-1 (simple graph) four times and then combining graphs A-1-1, and converting a graph obtained by the combination into a loop graph. In this case, graph A-3-3 and graph B-3 are the same graph. Then, graph A-3-3 and graph B-3 are represented by an Ising model equation and solved by the annealing machine as a maximum independent set problem in a conflict graph. Then, 64 atoms are found as common atoms, and the degree of similarity obtained by Formula (1-1) described previously is 1.00 (FIG. 21C).

<Third embodiment>

A crystalline material analysis device according to a third embodiment of the disclosed technique includes a graph creation unit, a graph replication unit, and an analysis unit.

A crystalline material analysis method according to the third embodiment of the disclosed technique includes a computer performing a graph creation process, a graph replication process, and an analysis process.

A crystalline material program according to the third embodiment of the disclosed technique includes causing a computer to perform graph creation, graph replication, and analysis.

The graph creation unit creates a graph having an intra-cell node and an intra-cell edge.

In the graph creation process, a graph having an intra-cell node and an intra-cell edge is created.

In the graph creation, a simple graph may be created.

In the graph creation, an extension graph may be created.

In the graph creation, a loop graph may be created.

In the graph creation, a graph is created for each of a first crystalline material and a second crystalline material.

The graph replication unit performs at least one of a first replication or a second replication.

In the graph replication process, at least one of the first replication or the second replication is performed.

In the first replication, a graph of a unit cell of the second crystalline material is replicated so that a guest lattice, which is a unit cell of the first crystalline material, fits in a host lattice created by connecting a plurality of unit cells of the second crystalline material.

In the second replication, a graph of a unit cell of the first crystalline material is replicated so that a guest lattice, which is a unit cell of the second crystalline material, fits in a host lattice created by connecting a plurality of unit cells of the first crystalline material.

It is sufficient if at least one of the first replication or the second replication is performed. Only the first replication may be performed, only the second replication may be performed, or both the first replication and the second replication may be performed.

Here, in a case where the unit cell of the first crystalline material and the unit cell of the second crystalline material differ in size, at least one of the first replication or the second replication is performed.

Note that the size of a unit cell is calculated from, for example, information regarding a bond distance between nodes at an edge.

The analysis unit analyzes a crystalline material.

In the analysis process, a crystalline material is analyzed.

In the analysis, a first comparison and a second comparison are performed.

The first comparison is a comparison between a graph constituted by a guest lattice of the first crystalline material and a graph constituted by a host lattice in which the guest lattice of the first crystalline material fits. The host lattice here is a host lattice constituted by one or a plurality of unit cells of the second crystalline material.

The second comparison is a comparison between a graph constituted by a guest lattice of the second crystalline material and a graph constituted by a host lattice in which the guest lattice of the second crystalline material fits. The host lattice here is a host lattice constituted by one or a plurality of unit cells of the first crystalline material.

The analysis is, for example, analysis of similarity between the first crystalline material and the second crystalline material.

A graph corresponding to a host lattice constituted by a plurality of unit cells is obtained by, for example, integrating replicated graphs. A graph corresponding to a host lattice constituted by a plurality of unit cells may be, for example, a graph in which a plurality of graphs obtained by replication is simply put together. In this case, there are no edges between the plurality of graphs.

Furthermore, a graph obtained by integrating replicated graphs may be, for example, a graph obtained by combining, by edges between graphs, a plurality of graphs obtained by replication. In this case, the edges between graphs are preferably added between the plurality of graphs so that the integrated graph matches the structure of the crystalline material.

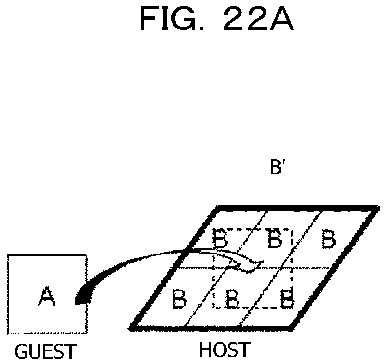

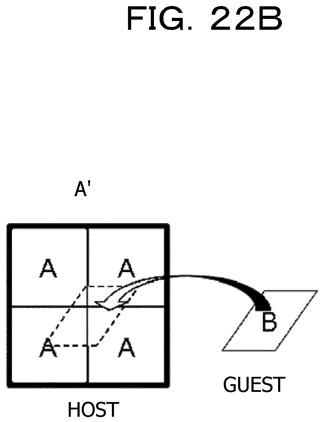

FIGs. 22A and 22B are schematic diagrams for illustrating replication of a graph in the third embodiment. FIG. 22A is a schematic diagram for illustrating the first replication. FIG. 22B is a schematic diagram for illustrating the second replication.

In FIGs. 22A and 22B, A represents a unit cell of the first crystalline material. B represents a unit cell of the second crystalline material.

As illustrated in FIG. 22A, in the first replication, a graph of unit cell B is replicated six times so that guest lattice A, which is unit cell A, fits in host lattice B' created by connecting the six replicated unit cells B. Here, the whole of the six replicated unit cells B is host lattice B'.

As illustrated in FIG. 22B, in the second replication, a graph of unit cell A is replicated four times so that guest lattice B, which is unit cell B, fits in host lattice A' created by connecting the four replicated unit cells A. Here, the whole of the four replicated unit cells A constitutes host lattice A'.

Here, an example of how to determine the number of times a unit cell is to be replicated to create a host lattice will be described. Here, a case of a plane will be described, and the same applies to a case of three dimensions.

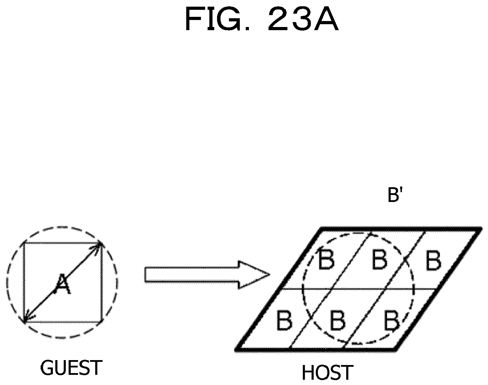

A case is considered where a graph of unit cell B is replicated so that guest lattice A fits in host lattice B' created by connecting unit cells B. In this case, as illustrated in FIG. 23A, the number of times unit cell B is to be replicated is determined so that a circle (a sphere in the case of three dimensions) having a diameter corresponding to the longest diagonal of guest lattice A fits in host lattice B' created by connecting unit cells B. Here, when considered in a plane, the circle is the smallest circle that can contain guest lattice A. When considered in three dimensions, the sphere is the smallest sphere that can contain guest lattice A. Furthermore, when considered in a plane, host lattice B' is the smallest size that can contain the circle. When considered in three dimensions, host lattice B' is the smallest size that can contain the sphere.

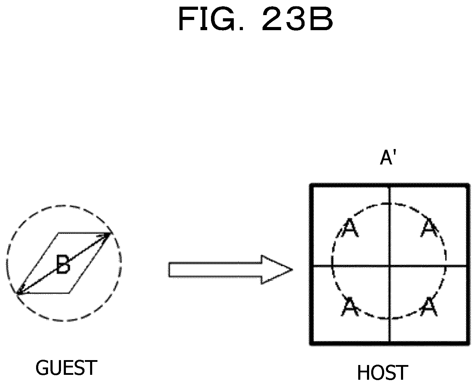

Furthermore, a case is considered where a graph of unit cell A is replicated so that guest lattice B fits in host lattice A' created by connecting unit cells A. In this case, as illustrated in FIG. 23B, the number of times unit cell A is to be replicated is determined so that a circle (a sphere in the case of three dimensions) having a diameter corresponding to the longest diagonal of guest lattice B fits in host lattice A' created by connecting unit cells A. Here, when considered in a plane, the circle is the smallest circle that can contain guest lattice B. When considered in three dimensions, the sphere is the smallest sphere that can contain guest lattice B. Furthermore, when considered in a plane, host lattice A' is the smallest size that can contain the circle. When considered in three dimensions, host lattice A' is the smallest size that can contain the sphere.

In the analysis, a graph of unit cell A (guest lattice A) of the first crystalline material and a graph of host lattice B' constituted by one or a plurality of unit cells B of the second crystalline material are compared. Moreover, in the analysis, a graph of unit cell B (guest lattice B) of the second crystalline material and a graph of host lattice A' constituted by one or a plurality of unit cells A of the first crystalline material are compared.

Here, using a graph constituted by unit cell A (guest lattice A) in FIG. 22A and a graph of host lattice B' constituted by six unit cells B in FIG. 22A, the degree of similarity between them is calculated.

Moreover, using a graph constituted by unit cell B (guest lattice B) in FIG. 22B and a graph of host lattice A' constituted by four unit cells A in FIG. 22B, the degree of similarity between them is calculated.

Here, in the calculation of the degree of similarity using a maximum independent set, the degree of similarity is obtained by, for example, the following Formula (2).

[Math. 4]

$$S(G_A, G_B) = \delta \max\left\{\frac{|V_C^{A'}|}{|V_A|}, \frac{|V_C^{B'}|}{|V_B|}\right\} + (1 - \delta) \min\left\{\frac{|V_C^{A'}|}{|V_A|}, \frac{|V_C^{B'}|}{|V_B|}\right\}$$

.. Formula (2)

Here, in the above Formula (2), $S(G_A, G_B)$ represents the degree of similarity between unit cell A of the first crystalline material expressed as a graph and unit cell B of the second crystalline material expressed as a graph, is represented as 0 to 1, and means that the closer to 1, the higher the degree of similarity.

Furthermore, $V_A$ represents the total number of node atoms in a guest lattice, which is unit cell A expressed as a graph, and $V_C^{A'}$ represents the number of node atoms included in a maximum independent set in a conflict graph, among node atoms in host lattice A' constituted by one or a plurality of unit cells A expressed as a graph. Note that the node atoms mean atoms at vertices of a molecule expressed as a graph.

Moreover, $V_B$ represents the total number of node atoms in a guest lattice, which is unit cell B expressed as a graph, and $V_C^B$ represents the number of node atoms included in the maximum independent set in the conflict graph, among node atoms in host lattice B' constituted by one or a plurality of unit cells B expressed as a graph.

$\delta$ denotes a number from 0 to 1.

Furthermore, in the above Formula (2), max{A, B} means to select A or B, whichever has a larger value, and min{A, B} means to select A or B, whichever has a smaller value.

Here, the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms constituting unit cell A or host lattice A' of the first crystalline material expressed as a graph and one of node atoms constituting unit cell B or host lattice B' of the second crystalline material expressed as a graph.

The above Formula (2) may be simplified to the following Formula (2-1). $\delta$ is 0.5.

[Math. 5]

$$(\text{Degree of similarity}) = \frac{1}{2}\left(\frac{AB'_{common}}{A_{all}} + \frac{A'B_{common}}{B_{all}}\right)$$

... Formula (2-1)

Here, $AB'_{common}$ represents the number of substructures common to guest lattice A and host lattice B'. $A'B_{common}$ represents the number of substructures common to guest lattice B and host lattice A'. $A_{all}$ represents the number of nodes in a graph of unit cell A, and $B_{all}$ represents the number of nodes in a graph of unit cell B.

In the case of the second embodiment, by replicating a graph so as to reduce the difference in the number of atoms (the number of nodes) between the two unit cells to be compared, it is possible to avoid a decrease in the degree of similarity caused by the difference in the number of atoms (the number of nodes). However, in a case where the numbers of unit cells after the replication do not match, the degree of similarity does not become 1.

On the other hand, in the case of the third embodiment, when Formula (2) or (2-1) for calculating the degree of similarity described previously is used, the maximum value of the degree of similarity is 1 even in a case where unit cell A and unit cell B differ in size.

Therefore, in the third embodiment, a decrease in the degree of similarity caused by the difference in the number of atoms (the number of nodes) of the two unit cells to be compared may be further avoided as compared with the second embodiment. As a result, the accuracy of analysis (e.g., the degree of similarity) may be further improved as compared with the second embodiment.

The graphing technique of the third embodiment is therefore useful for analysis of a crystalline material using computer technology.

Note that, for example, combinations of a guest lattice and a host lattice in the third embodiment may be classified into the following combinations, for example, depending on the structure of the graph.

- 3-1: The graph of the guest lattice is a simple graph.

  -- 3-1-1: The graph of the host lattice is a simple graph.
  -- 3-1-2: The graph of the host lattice is an extension graph.
  -- 3-1-3: The graph of the host lattice is a loop graph.

- 3-2: The graph of the guest lattice is an extension graph.

  -- 3-2-1: The graph of the host lattice is a simple graph.
  -- 3-2-2: The graph of the host lattice is an extension graph.
  -- 3-2-3: The graph of the host lattice is a loop graph.

- 3-3: The graph of the guest lattice is a loop graph.

  -- 3-2-1: The graph of the host lattice is a simple graph.
  -- 3-2-2: The graph of the host lattice is an extension graph.
  -- 3-2-3: The graph of the host lattice is a loop graph.

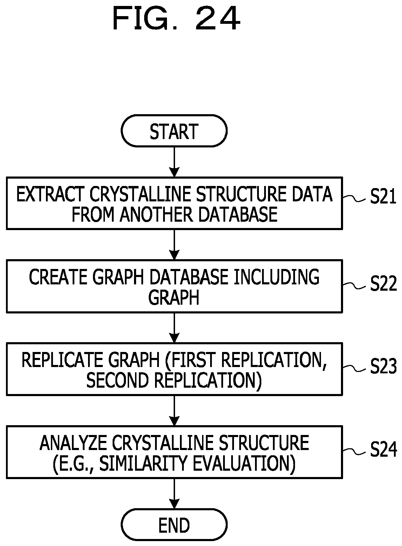

The crystalline material analysis method in the third embodiment will be briefly described with reference to a flowchart illustrated in FIG. 24.

First, crystalline structure data is extracted from another database (S21). The other database is not particularly limited and can be appropriately selected depending on a purpose. Examples thereof include an inorganic crystal structure database (ICSD) and the like. Note that the number of pieces and the types of crystalline structure data to be extracted are not particularly limited and can be appropriately selected depending on a purpose. For example, only crystalline structure data on chemicals having a specific element may be extracted.

Next, using the extracted crystalline structure data, a graph is created by graphing according to the technique of the present application, and a graph database including the graph is created (S22).

Next, the created graph is replicated (S23). In replicating the graph here, at least one of a first replication or a second replication is performed. In the first replication, a graph of a unit cell of the second crystalline material is replicated so that a guest lattice, which is a unit cell of the first crystalline material, fits in a host lattice created by connecting a plurality of unit cells of the second crystalline material. In the second replication, a graph of a unit cell of the first crystalline material is replicated so that a guest lattice, which is a unit cell of the second crystalline material, fits in a host lattice created by connecting a plurality of unit cells of the first crystalline material.

Next, the crystalline materials are analyzed (S24). In the analysis, a first comparison and a second comparison are performed. The first comparison is a comparison between a graph constituted by a guest lattice of the first crystalline material and a graph constituted by a host lattice in which the guest lattice of the first crystalline material fits. The host lattice here is a host lattice constituted by one or a plurality of unit cells of the second crystalline material. The second comparison is a comparison between a graph constituted by a guest lattice of the second crystalline material and a graph constituted by a host lattice in which the guest lattice of the second crystalline material fits. The host lattice here is a host lattice constituted by one or a plurality of unit cells of the first crystalline material. Examples of the analysis include similarity evaluation. The similarity evaluation is performed by, for example, solving, with the annealing machine or the like, a maximum independent set problem in a conflict graph represented by an Ising model equation.

Thus, it is possible to evaluate similarity in crystalline structure, which is one of analyses of a crystalline structure.

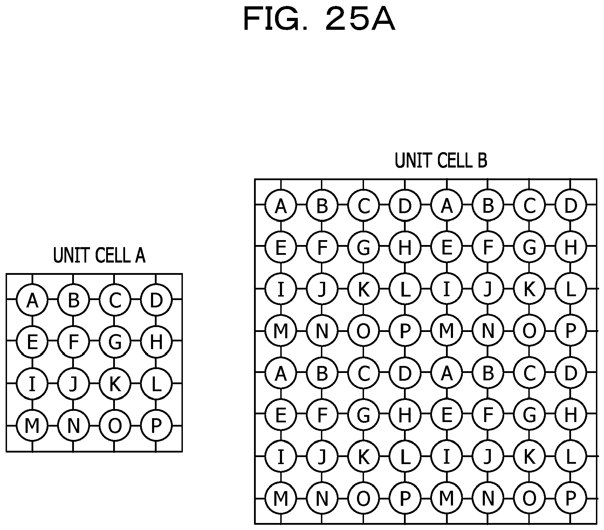

Here, a working example of the third embodiment will be described with reference to FIGs. 25A to 25C.

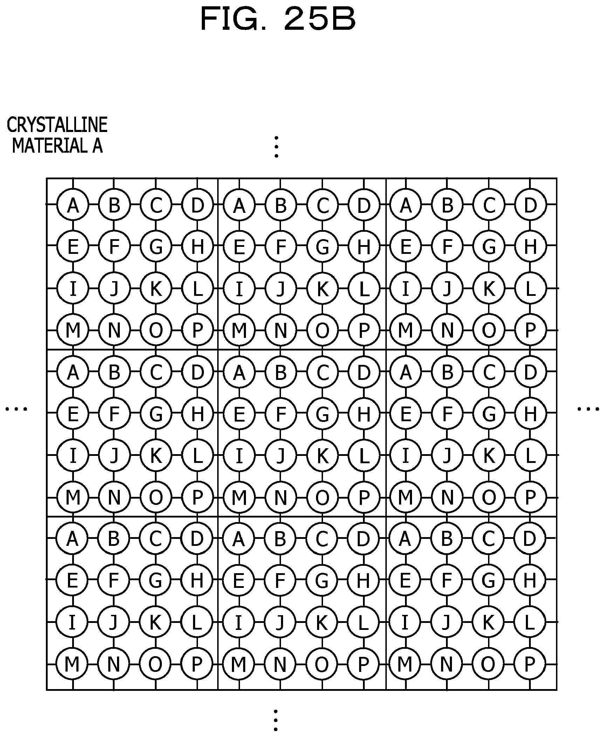

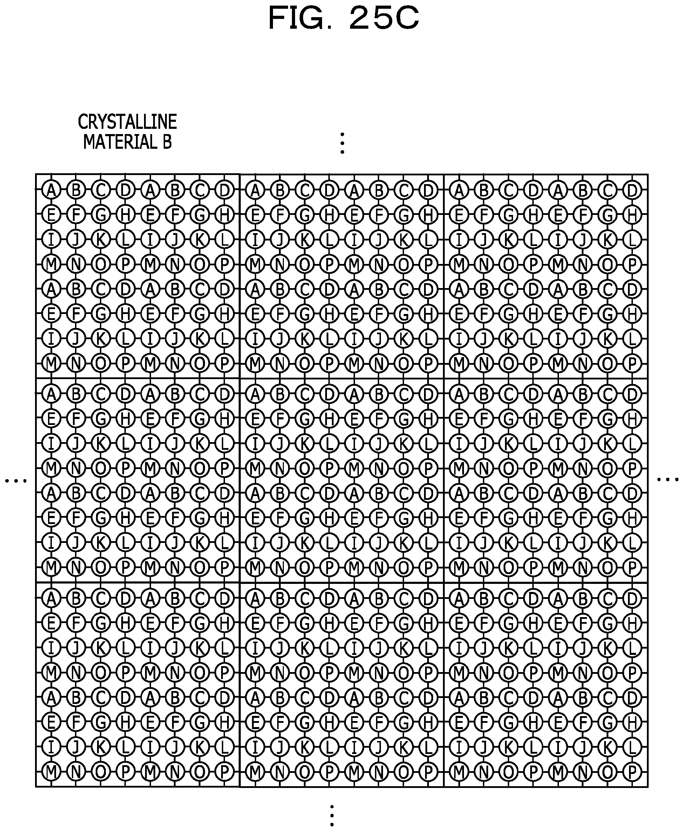

In this working example, the degree of similarity between crystalline material A represented by unit cell A and crystalline material B represented by unit cell B illustrated in FIG. 25A is obtained. Crystalline material A represented by unit cell A has a structure in which unit cell A is repeated as illustrated in FIG. 25B. Crystalline material B represented by unit cell B has a structure in which unit cell B is repeated as illustrated in FIG. 25C. Unit cell A and unit cell B are constituted by 16 distinct types of atoms A to P.

Using a graph created from unit cell A and a graph created from unit cell B, a maximum independent set problem in a conflict graph is represented by an Ising model equation and solved by the annealing machine. Then, the degree of similarity between the crystalline material of unit cell A and the crystalline material of unit cell B obtained by the above Formula (2-1) is as shown in the following Table 1.

[Table 1]

| Mode | Guest lattice | Host lattice | Degree of similarity | Guest lattice A Host lattice B' | | Guest lattice B Host lattice A' | |
|---|---|---|---|---|---|---|---|
| | | | | The number of unit cells B | The number of bits | The number of unit cells A | The number of bits |
| 3-1-1 | Simple graph | Simple graph | 1.000 | One | 64 | Nine | 576 |
| 3-1-2 | Simple graph | Extension graph | 1.000 | One | 96 | Nine | 864 |
| 3-1-3 | Simple graph | Loop graph | 1.000 | One | 64 | Nine | 576 |
| 3-2-1 | Extension gr a ph | Simple graph | 0. 688 | One | 128 | Nine | 768 |
| 3-2-2 | Extension graph | Extension graph | 0.792 | One | 208 | Nine | 1200 |
| 3-2-3 | Extension graph | Loop graph | 0.792 | One | 128 | Nine | 768 |
| 3-3-1 | Loop graph | Simple graph | 0.758 | One | 64 | Nine | 576 |
| 3-3-2 | Loop graph | Extension graph | 0.758 | One | 96 | Nine | 864 |
| 3-3-3 | Loop graph | Loop graph | 0.758 | One | 64 | Nine | 576 |

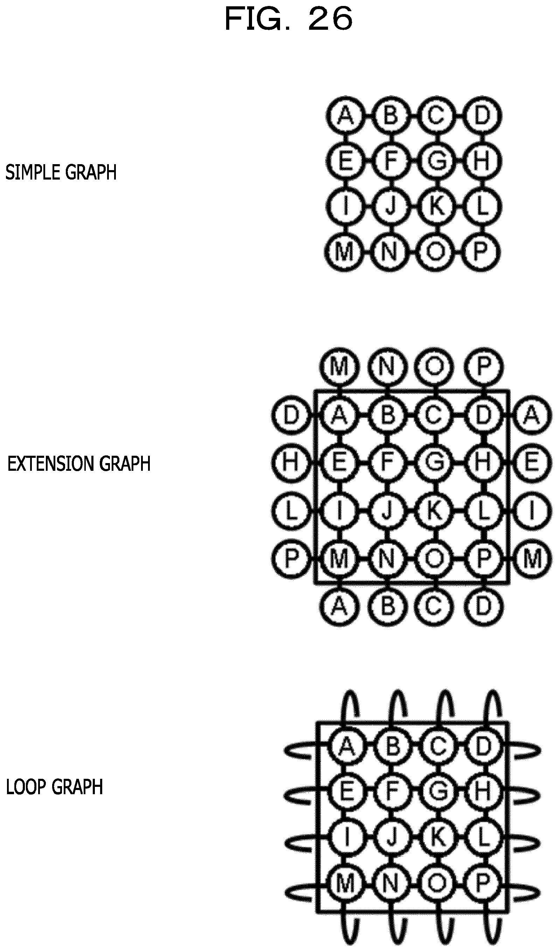

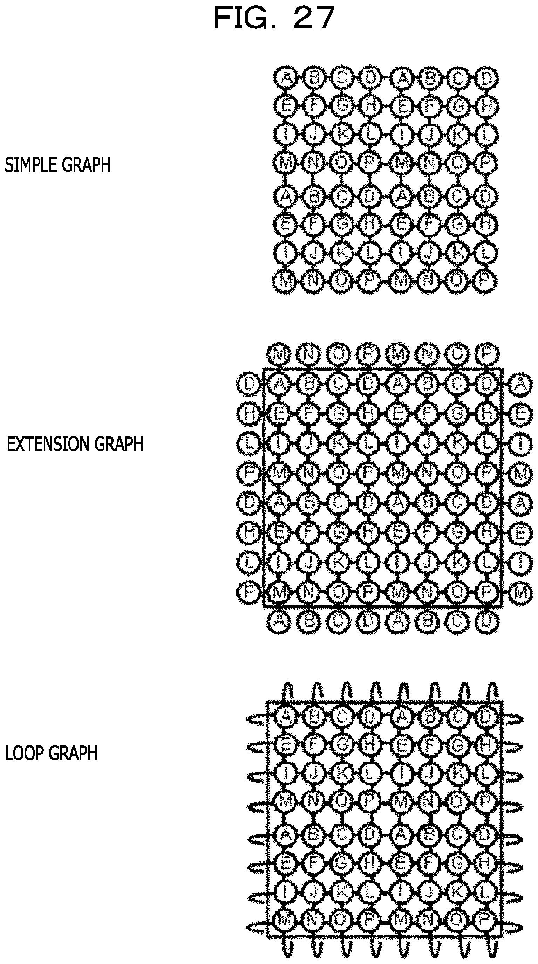

Note that, here, FIG. 26 illustrates a simple graph, an extension graph, and a loop graph in a case where unit cell A is used as guest lattice A. FIG. 27 illustrates a simple graph, an extension graph, and a loop graph in a case where unit cell B is used as guest lattice B.

Note that the extension graphs do not have any edge between extension nodes.

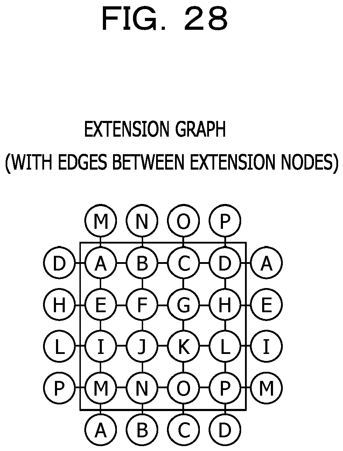

Moreover, Table 2 shows results of obtaining the degree of similarity in a similar manner to Table 1 for cases (mode 3-2'-1, 3-2'-2, and 3-2'-3) in which graphs created from unit cell A in modes 3-2-1, 3-2-2, and 3-2-3 shown in Table 1 are extension graphs having edges between extension nodes. Note that FIG. 28 illustrates a schematic diagram in a case where a graph created from unit cell A is an extension graph having edges between extension nodes. A comparison between mode 3-2-1 and mode 3-2'-1, a comparison between mode 3-2-2 and mode 3-2'-2, and a comparison between mode 3-2-3 and mode 3-2'-3 indicate that the degree of similarity becomes higher when an extension graph having edges between extension nodes is used.

[Table 2]

| Mode | Guest lattice | Host lattice | Degree of similarity | Guest lattice A Host lattice B' | | Guest lattice B Host lattice A' | |
|---|---|---|---|---|---|---|---|
| | | | | The number of unit cells B | The number of bits | The number of unit cells A | The number of bits |
| 3-2-1 | Extension graph | Simple graph | 0.688 | One | 128 | Nine | 768 |
| 3-2-2 | Extension graph | Extension graph | 0.792 | One | 208 | Nine | 1200 |
| 3-2-3 | Extension graph | Loop graph | 0.792 | One | 128 | Nine | 768 |
| 3-2'-1 | Extension graph | Simple graph | 0.792 | One | 128 | Nine | 768 |

(continued)

| Mode | Guest lattice | Host lattice | Degree of similarity | Guest lattice A Host lattice B' | | Guest lattice B Host lattice A' | |
|---|---|---|---|---|---|---|---|
| | | | | The number of unit cells B | The number of bits | The number of unit cells A | The number of bits |
| 3-2'-2 | Extension graph | Extension graph | 1.000 | One | 208 | Nine | 1200 |
| 3-2'-3 | Extension graph | Loop graph | 1.000 | One | 128 | Nine | 768 |

The following statements will be useful in understanding aspects of embodiments:

10. A crystalline material analysis device comprising:

> a graph creation unit that creates a graph that has intra-cell nodes that indicate atoms in one unit cell of a crystalline material and an intra-cell edge that indicates a chemical bond between atoms in the unit cell;
> a graph replication unit that generates a replicated graph by replicating the graph; and
> an analysis unit that analyzes the crystalline material by using the replicated graph.

11. The crystalline material analysis device according to statement 10, wherein

> the graph creation unit creates a first graph of a unit cell of a first crystalline material as the graph and a second graph of a unit cell of a second crystalline material as the graph,
> the graph replication unit replicates at least one of the first graph or the second graph, and
> the analysis unit analyzes similarity between the first crystalline material and the second crystalline material by using any one of the first graph or a first integrated graph obtained by integrating, with one another, a plurality of first replicated graphs generated by replicating the first graph, and any one of the second graph or a second integrated graph obtained by integrating, with one another, a plurality of second replicated graphs generated by replicating the second graph.

12. The crystalline material analysis device according to statement 11, wherein

> the analysis of similarity involves solving, with an annealing machine, a maximum independent set problem in a conflict graph represented by an Ising model equation,
> in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

13. The crystalline material analysis device according to statement 11 or 12, wherein
the replication of at least one of the first graph or the second graph is performed in such a way as to minimize an absolute value of a difference between the total number of nodes in a plurality of the first graphs after the replication and the total number of nodes in a plurality of the second graphs after the replication.

14. A crystalline material analysis method executed by a computer, the method comprising:

> a graph creation process of creating a graph that has intra-cell nodes that indicate atoms in one unit cell of a crystalline material and an intra-cell edge that indicates a chemical bond between atoms in the unit cell;
> a graph replication process of generating a replicated graph by replicating the graph; and
> an analysis process of analyzing the crystalline material by using the replicated graph.

15. The crystalline material analysis method according to statement 14, wherein

> in the graph creation process, a first graph of a unit cell of a first crystalline material as the graph and a second graph of a unit cell of a second crystalline material as the graph are created,

in the graph replication process, at least one of the first graph or the second graph is replicated, and
in the analysis process, similarity between the first crystalline material and the second crystalline material is analyzed by using any one of the first graph or a first integrated graph obtained by integrating, with one another, a plurality of first replicated graphs generated by replicating the first graph, and any one of the second graph or a second integrated graph obtained by integrating, with one another, a plurality of second replicated graphs generated by replicating the second graph.

16. The crystalline material analysis method according to statement 15, wherein

the analysis of similarity involves solving, with an annealing machine, a maximum independent set problem in a conflict graph represented by an Ising model equation,
in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

17. The crystalline material analysis method according to statement 15 or 16, wherein
the replication of at least one of the first graph or the second graph is performed in such a way as to minimize an absolute value of a difference between the total number of nodes in a plurality of the first graphs after the replication and the total number of nodes in a plurality of the second graphs after the replication.

18. A computer-readable recording medium having stored therein a program for causing a computer to execute a crystalline material analysis process comprising:

creating a graph that has intra-cell nodes that indicate atoms in one unit cell of a crystalline material and an intra-cell edge that indicates a chemical bond between atoms in the unit cell;
generating a replicated graph by replicating the graph; and
analyzing the crystalline material by using the replicated graph.

19. The computer-readable recording medium according to statement 18, wherein

in the graph creation, a first graph of a unit cell of a first crystalline material as the graph and a second graph of a unit cell of a second crystalline material as the graph are created,
in the graph replication, at least one of the first graph or the second graph is replicated, and
in the analysis, similarity between the first crystalline material and the second crystalline material is analyzed by using any one of the first graph or a first integrated graph obtained by integrating, with one another, a plurality of first replicated graphs generated by replicating the first graph, and any one of the second graph or a second integrated graph obtained by integrating, with one another, a plurality of second replicated graphs generated by replicating the second graph.

20. The computer-readable recording medium according to statement 19, wherein

the analysis of similarity involves solving, with an annealing machine, a maximum independent set problem in a conflict graph represented by an Ising model equation,
in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

21. The computer-readable recording medium according to statement 19 or 20, wherein
the replication of at least one of the first graph or the second graph is performed in such a way as to minimize an absolute value of a difference between the total number of nodes in a plurality of the first graphs after the replication and the total number of nodes in a plurality of the second graphs after the replication.

22. A crystalline material analysis device comprising:

a graph creation unit that creates a graph that has intra-cell nodes that indicate atoms in one unit cell of a crystalline material and an intra-cell edge that indicates a chemical bond between atoms in the unit cell for a first crystalline material and a second crystalline material;

a graph replication unit that performs at least one of a first replication in which a graph of a unit cell of the second crystalline material is replicated in such a way that a guest lattice, which is a unit cell of the first crystalline material, fits in a host lattice created by connecting a plurality of the unit cells of the second crystalline material, or a second replication in which a graph of the unit cell of the first crystalline material is replicated in such a way that a guest lattice, which is the unit cell of the second crystalline material, fits in a host lattice created by connecting a plurality of the unit cells of the first crystalline material; and

an analysis unit that analyzes the crystalline materials by performing a first comparison between a graph constituted by the guest lattice of the first crystalline material and a graph constituted by a host lattice constituted by one or a plurality of the unit cells of the second crystalline material in which the guest lattice of the first crystalline material fits, and also performing a second comparison between a graph constituted by the guest lattice of the second crystalline material and a graph constituted by a host lattice constituted by one or a plurality of the unit cells of the first crystalline material in which the guest lattice of the second crystalline material fits.

23. The crystalline material analysis device according to statement 22, wherein

the first replication replicates the graph of the unit cell of the second crystalline material in such a way that a sphere that has a diameter corresponding to the longest diagonal of the guest lattice of the first crystalline material fits in the host lattice, and

the second replication replicates the graph of the unit cell of the first crystalline material in such a way that a sphere that has a diameter corresponding to the longest diagonal of the guest lattice of the second crystalline material fits in the host lattice.

24. The crystalline material analysis device according to statement 22 or 23, wherein
the analysis unit analyzes similarity between the first crystalline material and the second crystalline material by using the first comparison and the second comparison.

25. The crystalline material analysis device according to statement 24, wherein

the analysis of similarity involves solving, with an annealing machine, a maximum independent set problem in a conflict graph represented by an Ising model equation,

in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

26. The crystalline material analysis device according to statement 25, wherein
the analysis unit uses the following Formula (2) to obtain a degree of similarity between the first crystalline material and the second crystalline material on the basis of the maximum independent set that has been searched for,
[Math. 1]

$$S(G_A, G_B) = \delta \max\left\{\frac{|V_C^A|}{|V_A|}, \frac{|V_C^B|}{|V_B|}\right\} + (1 - \delta)\min\left\{\frac{|V_C^A|}{|V_A|}, \frac{|V_C^B|}{|V_B|}\right\}$$

$$\text{.. Formula (2)}$$

where, in the above Formula (2),
the $G_A$ represents the first crystalline material,
the $G_B$ represents the second crystalline material,
the $S(G_A, G_B)$ represents a degree of similarity between the first crystalline material and the second crystalline material expressed as a graph, is represented as 0 to 1, and means that the closer to 1, the higher the degree of similarity,
the $V_A$ represents the total number of the node atoms in the guest lattice, which is the unit cell of the first crystalline material expressed as a graph,
the $V_C^A$ represents the number of the node atoms included in the maximum independent set of the conflict graph among the node atoms in the host lattice constituted by one or a plurality of the unit cells of the first crystalline material expressed as a graph,

the $V_B$ represents the total number of the node atoms in the guest lattice, which is the unit cell of the second crystalline material expressed as a graph,

the $V_c^B$ represents the number of the node atoms included in the maximum independent set of the conflict graph among the node atoms in the host lattice constituted by one or a plurality of the unit cells of the second crystalline material expressed as a graph, and

the $\delta$ denotes a number from 0 to 1.

27. A crystalline material analysis method executed by a computer, the method comprising:

a graph creation process of creating a graph that has intra-cell nodes that indicate atoms in one unit cell of a crystalline material and an intra-cell edge that indicates a chemical bond between atoms in the unit cell for a first crystalline material and a second crystalline material;

a graph replication process of performing at least one of a first replication in which a graph of a unit cell of the second crystalline material is replicated in such a way that a guest lattice, which is a unit cell of the first crystalline material, fits in a host lattice created by connecting a plurality of the unit cells of the second crystalline material, or a second replication in which a graph of the unit cell of the first crystalline material is replicated in such a way that a guest lattice, which is the unit cell of the second crystalline material, fits in a host lattice created by connecting a plurality of the unit cells of the first crystalline material; and

an analysis process of analyzing the crystalline materials by performing a first comparison between a graph constituted by the guest lattice of the first crystalline material and a graph constituted by a host lattice constituted by one or a plurality of the unit cells of the second crystalline material in which the guest lattice of the first crystalline material fits, and also performing a second comparison between a graph constituted by the guest lattice of the second crystalline material and a graph constituted by a host lattice constituted by one or a plurality of the unit cells of the first crystalline material in which the guest lattice of the second crystalline material fits.

28. The crystalline material analysis method according to statement 27, wherein

the first replication replicates the graph of the unit cell of the second crystalline material in such a way that a sphere that has a diameter corresponding to the longest diagonal of the guest lattice of the first crystalline material fits in the host lattice, and

the second replication replicates the graph of the unit cell of the first crystalline material in such a way that a sphere that has a diameter corresponding to the longest diagonal of the guest lattice of the second crystalline material fits in the host lattice.

29. The crystalline material analysis method according to statement 27 or 28, wherein
in the analysis process, similarity between the first crystalline material and the second crystalline material is analyzed by using the first comparison and the second comparison.

30. The crystalline material analysis method according to statement 29, wherein

the analysis of similarity involves solving, with an annealing machine, a maximum independent set problem in a conflict graph represented by an Ising model equation,
in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

31. The crystalline material analysis method according to statement 30, wherein
in the analysis process, the following Formula (2) is used to obtain a degree of similarity between the first crystalline material and the second crystalline material on the basis of the maximum independent set that has been searched for,
[Math. 2]

$$S(G_A, G_B) = \delta \max \left\{ \frac{|V_c^A|}{|V_A|}, \frac{|V_c^B|}{|V_B|} \right\} + (1 - \delta) \min \left\{ \frac{|V_c^A|}{|V_A|}, \frac{|V_c^B|}{|V_B|} \right\}$$

`.. Formula (2)`

where, in the above Formula (2),

the $G_A$ represents the first crystalline material,

the $G_B$ represents the second crystalline material,

the $S(G_A, G_B)$ represents a degree of similarity between the first crystalline material and the second crystalline material expressed as a graph, is represented as 0 to 1, and means that the closer to 1, the higher the degree of similarity,

the $V_A$ represents the total number of the node atoms in the guest lattice, which is the unit cell of the first crystalline material expressed as a graph,

the $V_c^A$ represents the number of the node atoms included in the maximum independent set of the conflict graph among the node atoms in the host lattice constituted by one or a plurality of the unit cells of the first crystalline material expressed as a graph,

the $V_B$ represents the total number of the node atoms in the guest lattice, which is the unit cell of the second crystalline material expressed as a graph,

the $V_c^B$ represents the number of the node atoms included in the maximum independent set of the conflict graph among the node atoms in the host lattice constituted by one or a plurality of the unit cells of the second crystalline material expressed as a graph, and

the $\delta$ denotes a number from 0 to 1.

32. A computer-readable recording medium having stored therein a program for causing a computer to execute a crystalline material analysis process comprising:

creating a graph that has intra-cell nodes that indicate atoms in one unit cell of a crystalline material and an intra-cell edge that indicates a chemical bond between atoms in the unit cell for a first crystalline material and a second crystalline material;

performing at least one of a first replication in which a graph of a unit cell of the second crystalline material is replicated in such a way that a guest lattice, which is a unit cell of the first crystalline material, fits in a host lattice created by connecting a plurality of the unit cells of the second crystalline material, or a second replication in which a graph of the unit cell of the first crystalline material is replicated in such a way that a guest lattice, which is the unit cell of the second crystalline material, fits in a host lattice created by connecting a plurality of the unit cells of the first crystalline material; and

analyzing the crystalline materials by performing a first comparison between a graph constituted by the guest lattice of the first crystalline material and a graph constituted by a host lattice constituted by one or a plurality of the unit cells of the second crystalline material in which the guest lattice of the first crystalline material fits, and also performing a second comparison between a graph constituted by the guest lattice of the second crystalline material and a graph constituted by a host lattice constituted by one or a plurality of the unit cells of the first crystalline material in which the guest lattice of the second crystalline material fits.

33. The computer-readable recording medium according to statement 32, wherein

the first replication replicates the graph of the unit cell of the second crystalline material in such a way that a sphere that has a diameter corresponding to the longest diagonal of the guest lattice of the first crystalline material fits in the host lattice, and

the second replication replicates the graph of the unit cell of the first crystalline material in such a way that a sphere that has a diameter corresponding to the longest diagonal of the guest lattice of the second crystalline material fits in the host lattice.

34. The computer-readable recording medium according to statement 32 or 33, wherein

in the analysis, similarity between the first crystalline material and the second crystalline material is analyzed by using the first comparison and the second comparison.

35. The computer-readable recording medium according to statement 34, wherein

the analysis of similarity involves solving, with an annealing machine, a maximum independent set problem in a conflict graph represented by an Ising model equation,

in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

36. The computer-readable recording medium according to statement 35, wherein
in the analysis, the following Formula (2) is used to obtain a degree of similarity between the first crystalline material and the second crystalline material on the basis of the maximum independent set that has been searched for,
[Math. 3]

$$S(G_A, G_B) = \delta \max\left\{\frac{|V_C^{A}|}{|V_A|}, \frac{|V_C^{B}|}{|V_B|}\right\} + (1 - \delta) \min\left\{\frac{|V_C^{A}|}{|V_A|}, \frac{|V_C^{B}|}{|V_B|}\right\} \quad \text{.. Formula (2)}$$

where, in the above Formula (2),
the $G_A$ represents the first crystalline material,
the $G_B$ represents the second crystalline material,
the $S(G_A, G_B)$ represents a degree of similarity between the first crystalline material and the second crystalline material expressed as a graph, is represented as 0 to 1, and means that the closer to 1, the higher the degree of similarity,
the $V_A$ represents the total number of the node atoms in the guest lattice, which is the unit cell of the first crystalline material expressed as a graph,
the $V_C^A$ represents the number of the node atoms included in the maximum independent set of the conflict graph among the node atoms in the host lattice constituted by one or a plurality of the unit cells of the first crystalline material expressed as a graph,
the $V_B$ represents the total number of the node atoms in the guest lattice, which is the unit cell of the second crystalline material expressed as a graph,
the $V_C^B$ represents the number of the node atoms included in the maximum independent set of the conflict graph among the node atoms in the host lattice constituted by one or a plurality of the unit cells of the second crystalline material expressed as a graph, and
the $\delta$ denotes a number from 0 to 1.

## Claims

1. A crystalline material analysis device comprising:

   a graph creation unit that creates a loop graph that has intra-cell nodes that indicate atoms in one unit cell of a crystalline material, an intra-cell edge that indicates a chemical bond between atoms in the unit cell, and a loop edge that indicates the chemical bond, which is virtual, between atom X in the unit cell and atom Y in the unit cell corresponding to atom Y' in an adjacent unit cell that is adjacent to the unit cell, in which the atom Y' has the chemical bond with the atom X; and
   an analysis unit that analyzes the crystalline material by using the graph.

2. The crystalline material analysis device according to claim 1, wherein

   the graph creation unit creates a first loop graph of a unit cell of a first crystalline material as the loop graph and a second loop graph of a unit cell of a second crystalline material as the loop graph, and
   the analysis unit analyzes similarity between the first crystalline material and the second crystalline material by using the first loop graph and the second loop graph.

3. The crystalline material analysis device according to claim 2, wherein

   the analysis of similarity involves solving, with an annealing machine, a maximum independent set problem in a conflict graph represented by an Ising model equation,
   in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

**4.** A crystalline material analysis method executed by a computer, the method comprising:

a graph creation process of creating a loop graph that has intra-cell nodes that indicate atoms in one unit cell of a crystalline material, an intra-cell edge that indicates a chemical bond between atoms in the unit cell, and a loop edge that indicates the chemical bond, which is virtual, between atom X in the unit cell and atom Y in the unit cell corresponding to atom Y' in an adjacent unit cell that is adjacent to the unit cell, in which the atom Y' has the chemical bond with the atom X; and

an analysis process of analyzing the crystalline material by using the loop graph.

**5.** The crystalline material analysis method according to claim 4, wherein

in the graph creation process, a first loop graph of a unit cell of a first crystalline material as the loop graph and a second loop graph of a unit cell of a second crystalline material as the loop graph are created, and

in the analysis process, similarity between the first crystalline material and the second crystalline material is analyzed by using the first loop graph and the second loop graph.

**6.** The crystalline material analysis method according to claim 5, wherein

the analysis of similarity involves solving, with an annealing machine, a maximum independent set problem in a conflict graph represented by an Ising model equation,

in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

**7.** A program for causing a computer to execute a crystalline material analysis process comprising:

creating a loop graph that has intra-cell nodes that indicate atoms in one unit cell of a crystalline material, an intra-cell edge that indicates a chemical bond between atoms in the unit cell, and a loop edge that indicates the chemical bond, which is virtual, between atom X in the unit cell and atom Y in the unit cell corresponding to atom Y' in an adjacent unit cell that is adjacent to the unit cell, in which the atom Y' has the chemical bond with the atom X; and

analyzing the crystalline material by using the loop graph.

**8.** The program according to claim 7, wherein

in the loop graph creation, a first loop graph of a unit cell of a first crystalline material as the loop graph and a second loop graph of a unit cell of a second crystalline material as the loop graph are created, and

in the analysis, similarity between the first crystalline material and the second crystalline material is analyzed by using the first loop graph and the second loop graph.

**9.** The program according to claim 8, wherein

the analysis of similarity involves solving, with an annealing machine, a maximum independent set problem in a conflict graph represented by an Ising model equation,

in which the conflict graph is created on the basis of a rule that an edge is created between two nodes when the nodes are compared and are not identical to each other, and no edge is created between two nodes when the nodes are compared and are identical to each other, in which each of the nodes is a combination of one of node atoms that constitute the unit cell of the first crystalline material expressed as a graph and one of node atoms that constitute the unit cell of the second crystalline material expressed as a graph.

# FIG. 1

```
                                                          ⌐10

        ⌐11                              ⌐14
┌──────────────────┐            ┌──────────────────┐
│   CONTROL UNIT    │◄────►◄────►│   DISPLAY UNIT    │
└──────────────────┘            └──────────────────┘

        ⌐12                              ⌐15
┌──────────────────┐            ┌──────────────────┐
│     MEMORY        │◄────►◄────►│    INPUT UNIT     │
└──────────────────┘            └──────────────────┘

        ⌐13                              ⌐16
┌──────────────────┐            ┌──────────────────┐
│  STORAGE UNIT     │◄────►◄────►│   OUTPUT UNIT     │
│  (HARD DISK)      │            └──────────────────┘
└──────────────────┘
                                         ⌐17
                                ┌──────────────────┐
                           ◄────►│  I/O INTERFACE    │
                                │      UNIT         │
                                └──────────────────┘
          18
```

# FIG. 2

# FIG. 3

```
                                                    ⌇30
┌─────────────────────────────────────────────────────┐
│                                                       │
│      ⌇11                          ⌇14                  │
│  ┌──────────────┐          ┌──────────────┐           │
│  │ CONTROL UNIT │ ◄──►│◄──► │ DISPLAY UNIT │           │
│  └──────────────┘          └──────────────┘           │
│      ⌇12                          ⌇15                  │
│  ┌──────────────┐          ┌──────────────┐           │
│  │    MEMORY    │ ◄──►│◄──► │  INPUT UNIT  │           │
│  └──────────────┘          └──────────────┘           │
│                                   ⌇16                  │
│                            ┌──────────────┐           │
│                       ◄──► │ OUTPUT UNIT  │           │
│                            └──────────────┘           │
│                                   ⌇17                  │
│                            ┌──────────────┐           │
│                       ◄──► │ I/O INTERFACE│           │
│                            │    UNIT      │           │
│                            └──────────────┘           │
│                                   ⌇19                  │
│                            ┌──────────────┐           │
│                       ◄──► │   NETWORK    │           │
│                            │INTERFACE UNIT│           │
│   18⌇                      └──────────────┘           │
└─────────────────────────────────────────────────────┘
```

CONTROL UNIT 11

MEMORY 12

DISPLAY UNIT 14

INPUT UNIT 15

OUTPUT UNIT 16

I/O INTERFACE UNIT 17

NETWORK INTERFACE UNIT 19

18

30

40

STORAGE UNIT (HARD DISK) 13

NETWORK INTERFACE UNIT 20

# FIG. 4

# FIG. 5

1B

5

GRAPH DATABASE

2

GRAPH CREATION UNIT

4

ANALYSIS UNIT

# FIG. 6

1C

┌─────────────────────┐
│ 2                   │
│ ┌─────────────────┐ │
│ │ GRAPH CREATION  │ │
│ │      UNIT       │ │
│ └─────────────────┘ │
│ 3                   │
│ ┌─────────────────┐ │
│ │     GRAPH       │ │
│ │ REPLICATION UNIT│ │
│ └─────────────────┘ │
│ 4                   │
│ ┌─────────────────┐ │
│ │  ANALYSIS UNIT  │ │
│ └─────────────────┘ │
└─────────────────────┘

# FIG. 7

# FIG. 8

# FIG. 9

# FIG. 1O

# FIG. 11

# FIG. 12

```
┌─────────────┐
│    START    │
└─────────────┘
       │
       ▼
┌──────────────────────────────────┐
│ EXTRACT CRYSTALLINE STRUCTURE DATA│ ～S1
│      FROM ANOTHER DATABASE        │
└──────────────────────────────────┘
       │
       ▼
┌──────────────────────────────────┐
│  CREATE GRAPH DATABASE INCLUDING  │ ～S2
│            LOOP GRAPH             │
└──────────────────────────────────┘
       │
       ▼
┌──────────────────────────────────┐
│     ANALYZE CRYSTALLINE STRUCTURE │ ～S3
│   (E.G., SIMILARITY EVALUATION)   │
└──────────────────────────────────┘
       │
       ▼
┌─────────────┐
│     END     │
└─────────────┘
```

# FIG. 13A

```
READ CRYSTALLINE MATERIAL DATA ─── S101
                    │
                    ▼
CALCULATE RELATIVE COORDINATES OF ALL ATOMS
        IN SINGLE UNIT CELL ─── S102
                    │
                    ▼
REPRESENT ALL ATOMS IN SINGLE UNIT CELL AS
        NODES OF GRAPH ─── S103
                    │
                    ▼
        EACH ATOM A ─── S104
                    │
                    ▼
        EACH ATOM B ─── S105
                    │
                    ▼
```

S106

IS DISTANCE BETWEEN A AND B
APPROPRIATE AS BOND DISTANCE?

YES

NO

S107

BOND NODES A AND B

```
        EACH ATOM B ─── S108
                    │
                    ▼
                   ( I )
```

FIG. 13B

(I)

EACH ATOM B' IN ADJACENT UNIT CELL — S109

IS THERE ALREADY BOND EQUIVALENT TO BOND BETWEEN A AND B'? — S110

NO

YES

IS DISTANCE BETWEEN A AND B' APPROPRIATE AS BOND DISTANCE? — S111

YES

NO

DOES NODE CORRESPONDING TO B' ALREADY EXIST? — S112

NO

YES

ADD ATOM B' AS EXTENSION NODE — S113

BOND NODES A AND B' — S114

EACH ATOM B' — S115

EACH ATOM A — S116

(II)

# FIG. 13C

```
        Ⅱ
        │
        ▼
┌─────────────────────────────┐
│   EACH EXTENSION NODE B'     │──── S117
└─────────────────────────────┘
        │
        ▼
┌─────────────────────────────┐
│ FIND NODE A' IN UNIT CELL    │
│      EQUIVALENT TO B'        │──── S118
└─────────────────────────────┘
        │
        ▼
┌─────────────────────────────┐
│       REGARD B' AS A'        │──── S119
└─────────────────────────────┘
        │
        ▼
┌─────────────────────────────┐
│   EACH EXTENSION NODE B'     │──── S120
└─────────────────────────────┘
        │
        ▼
     ( END )
```

FIG. 14A

# FIG. 14B

A-1

COMMON ATOMS:
SIX ATOMS
A,C,F,H,J,L

DEGREE OF SIMILARITY:
0.50

B-1

# FIG. 14C

A-2

B-2

COMMON ATOMS:
12 ATOMS

DEGREE OF SIMILARITY:
1.00

# FIG. 15A

# FIG. 15B

# FIG. 15C

# FIG. 16A

200A

# FIG. 16B

200B

# FIG. 17

```
          ┌─────────────┐
          │    START    │
          └─────────────┘
                 │
                 ▼
┌───────────────────────────────────┐
│ EXTRACT CRYSTALLINE STRUCTURE DATA │ ～ S11
│       FROM ANOTHER DATABASE        │
└───────────────────────────────────┘
                 │
                 ▼
┌───────────────────────────────────┐
│    CREATE GRAPH DATABASE INCLUDING │ ～ S12
│              GRAPH                 │
└───────────────────────────────────┘
                 │
                 ▼
┌───────────────────────────────────┐
│          REPLICATE GRAPH          │ ～ S13
└───────────────────────────────────┘
                 │
                 ▼
┌───────────────────────────────────┐
│     ANALYZE CRYSTALLINE STRUCTURE  │ ～ S14
│     (E.G., SIMILARITY EVALUATION)  │
└───────────────────────────────────┘
                 │
                 ▼
          ┌─────────────┐
          │     END     │
          └─────────────┘
```

# FIG. 18A

UNIT CELL B

UNIT CELL A

# FIG. 18B

CRYSTALLINE
MATERIAL A

# FIG. 18C

CRYSTALLINE
MATERIAL B

# FIG. 19A

GRAPH A-1-1
(SIMPLE GRAPH)

GRAPH B-1
(SIMPLE GRAPH)

COMMON ATOMS:
16 ATOMS

DEGREE OF
SIMILARITY: 0.625

# FIG. 19B

GRAPH A-1-2
(INTEGRATED GRAPH)

COMMON ATOMS:
50 ATOMS

DEGREE OF SIMILARITY:
0.78

GRAPH B-1
(SIMPLE GRAPH)

NO EDGES BETWEEN GRAPHS

# FIG. 19C

GRAPH A-1-3
(INTEGRATED GRAPH)

WITH EDGES BETWEEN GRAPHS

COMMON ATOMS:
64 ATOMS

DEGREE OF SIMILARITY:
1.00

GRAPH B-1
(SIMPLE GRAPH)

# FIG. 20A

GRAPH A-2-1
(EXTENSION GRAPH)

GRAPH B-2
(EXTENSION GRAPH)

COMMON ATOMS:
28 ATOMS

DEGREE OF SIMILARITY:
0.583

## FIG. 20B

GRAPH A-2-2
(INTEGRATED GRAPH)

GRAPH B-2
(EXTENSION GRAPH)

COMMON ATOMS:
82 ATOMS

DEGREE OF
SIMILARITY: 0.75

NO EDGES BETWEEN GRAPHS

# FIG. 20C

GRAPH A-2-3
(INTEGRATED GRAPH)

GRAPH B-2
(EXTENSION GRAPH)

COMMON ATOMS:
96 ATOMS

DEGREE OF
SIMILARITY: 1.00

WITH EDGES BETWEEN GRAPHS

# FIG. 21A

GRAPH A-3-1
(LOOP GRAPH)

GRAPH B-3
(LOOP GRAPH)

COMMON ATOMS:
11 ATOMS

DEGREE OF SIMILARITY:
0.42

FIG. 21B

GRAPH A-3-2
(INTEGRATED GRAPH)

GRAPH B-3 (LOOP GRAPH)

COMMON ATOMS:
44 ATOMS

DEGREE OF SIMILARITY:
0.68

NO EDGES BETWEEN GRAPHS

# FIG. 21C

GRAPH A-3-3
(INTEGRATED GRAPH)

GRAPH B-3 (LOOP GRAPH)

COMMON ATOMS:
64 ATOMS

DEGREE OF SIMILARITY:
1.00

WITH EDGES BETWEEN GRAPHS

# FIG. 22A

# FIG. 22B

A'

A    A

A    A

HOST

B

GUEST

# FIG. 23A

B'

GUEST

HOST

# FIG. 23B

GUEST

HOST

# FIG. 24

```
        ┌─────────────┐
        │    START    │
        └─────────────┘
               │
               ▼
┌─────────────────────────────────┐
│ EXTRACT CRYSTALLINE STRUCTURE DATA │─～ S21
│     FROM ANOTHER DATABASE         │
└─────────────────────────────────┘
               │
               ▼
┌─────────────────────────────────┐
│  CREATE GRAPH DATABASE INCLUDING  │─～ S22
│            GRAPH                  │
└─────────────────────────────────┘
               │
               ▼
┌─────────────────────────────────┐
│  REPLICATE GRAPH (FIRST REPLICATION, │─～ S23
│     SECOND REPLICATION)           │
└─────────────────────────────────┘
               │
               ▼
┌─────────────────────────────────┐
│   ANALYZE CRYSTALLINE STRUCTURE   │─～ S24
│   (E.G., SIMILARITY EVALUATION)   │
└─────────────────────────────────┘
               │
               ▼
        ┌─────────────┐
        │     END     │
        └─────────────┘
```

# FIG. 25A

UNIT CELL B

UNIT CELL A

# FIG. 25B

CRYSTALLINE
MATERIAL A

# FIG. 25C

CRYSTALLINE
MATERIAL B

# FIG. 26

SIMPLE GRAPH

EXTENSION GRAPH

LOOP GRAPH

# FIG. 27

SIMPLE GRAPH

EXTENSION GRAPH

LOOP GRAPH

# FIG. 28

EXTENSION GRAPH

(WITH EDGES BETWEEN EXTENSION NODES)

# FIG. 29

# FIG. 30

Europäisches
Patentamt

European
Patent Office

Office européen
des brevets

# EUROPEAN SEARCH REPORT

**Application Number**

EP 21 15 2198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOMMA KOICHI ET AL: "VESTA 3 for three-dimensional visualization of crystal, volumetric and morphology data", JOURNAL OF APPLIED CRYSTALLOGRAPHY., vol. 44, no. 6, 29 October 2011 (2011-10-29), pages 1272-1276, XP055812623, DK ISSN: 0021-8898, DOI: 10.1107/S0021889811038970 * abstract; figure 1 * * page 1272, column 1, paragraph 5 - column 2, paragraph 4; figures 2, 3, 7 * * page 1273, column 2, paragraph 2 * | 1-9 | INV. G16C20/80 G16C60/00 |
| X | HERNANDEZ MARITZA ET AL: "A Quantum-Inspired Method for Three-Dimensional Ligand-Based Virtual Screening", JOURNAL OF CHEMICAL INFORMATION AND MODELING, vol. 59, no. 10, 18 October 2019 (2019-10-18), pages 4475-4485, XP055793578, US ISSN: 1549-9596, DOI: 10.1021/acs.jcim.9b00195 Retrieved from the Internet: URL:https://pubs.acs.org/doi/pdf/10.1021/acs.jcim.9b00195> * abstract; figures 1,3 * * page 4476, column 2, paragraph 3 - page 4478, column 1, paragraph 1 * | 1-9 | |

**TECHNICAL FIELDS SEARCHED (IPC)**

G16C
G06F

The present search report has been drawn up for all claims

| Place of search | Date of completion of the search | Examiner |
|---|---|---|
| The Hague | 10 June 2021 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

**REFERENCES CITED IN THE DESCRIPTION**

**Patent documents cited in the description**

- JP 2011170444 A

**Non-patent literature cited in the description**

- **MARITZA HERNANDEZ ; ARMAN ZARIBAFIYAN ; MALIHEH ARAMON ; MOHAMMAD NAGHIBI.** *A Novel Graph-based Approach for Determining Molecular Similarity, https://arxiv.org/pdf/1601.06693.pdf*

- **MARITZA HERNANDEZ ; ARMAN ZARIBAFIYAN ; MALIHEH ARAMON ; MOHAMMAD NAGHIBI.** A Novel Graph-based Approach for Determining Molecular Similarity. *ArXiv: 1601.06693, https://arxiv.org/pdf/1601.06693.pdf*